# EUROPEAN PATENT SPECIFICATION

(11) **EP 2 473 053 B1**
(45) Date of publication and mention of the grant of the patent: **22.04.2015**
(21) Application number: 10814504.6
(22) Date of filing: 02.09.2010
(51) Int. Cl.: C07D 473/04, A61K 31/522, A61P 13/12

(54) **SUBSTITUTED XANTHINE DERIVATIVES**
SUBSTITUIERTE XANTHINDERIVATE
DÉRIVÉS DE XANTHINE SUBSTITUÉS

(30) Priority: 02.09.2009 US 239342 P; 01.09.2010 US 873991
(43) Date of publication of application: 11.07.2012
(73) Proprietor: Concert Pharmaceuticals Inc., Lexington, MA 02421 (US)
(72) Inventor: TUNG, Roger D., Lexington Massachusetts 02420 (US); LIU, Julie F., Lexington Massachusetts 02420 (US); HARBESON, Scott L., Cambridge Massachusetts 02140 (US)
(74) Representative: Duncan, Garreth Andrew
(86) International application number: PCT/US2010/047708
(87) International publication number: WO 2011/028922

(56) References cited:
- US-A- 5 112 827
- US-A- 5 780 476
- US-A1- 2005 107 420
- US-A1- 2009 239 886
- RAOUL ET AL: "A novel drug interaction between the quinolone antibiotic ciprofloxacin and a chiral metabolite of pentoxifylline", BIOCHEMICAL PHARMACOLOGY, PERGAMON, OXFORD, GB, vol. 74, no. 4, 12 July 2007 (2007-07-12), pages 639-646, XP022149875, ISSN: 0006-2952, DOI: 10.1016/J.BCP.2007.05.013
- BUTEAU K C: "Deuterated Drugs: Unexpectedly Nonobvious?", JOURNAL OF HIGH TECHNOLOGY LAW, SUFFOLK UNIVERSITY LAW SCHOOL, US , vol. X, no. 1 1 January 2009 (2009-01-01), pages 22-74, XP002636702, ISSN: 1536-7983 Retrieved from the Internet: URL:http://www.law.suffolk.edu/highlights/ stuorgs/jhtl/docs/pdf/Buteau_10JHTL1.pdf [retrieved on 2009-01-01]
- LIN ET AL.: 'The Renoprotective Potential of Pentoxifylline in Chronic Kidney Disease.' J CHIN MED ASSOC vol. 68, no. 3, 2005, pages 99 - 105, XP026304647

## Description

### RELATED APPLICATIONS

This application claims the benefit of priority of U.S. Patent Application Number 12/873,991, filed September 1, 2010 and U.S. Provisional Application Number 61/239,342, filed September 2, 2009.

### BACKGROUND OF THE INVENTION

Many current medicines suffer from poor absorption, distribution, metabolism and/or excretion (ADME) properties that prevent their wider use. Poor ADME properties are also a major reason for the failure of drug candidates in clinical trials. While formulation technologies and prodrug strategies can be employed in some cases to improve certain ADME properties, these approaches have failed to overcome the inherent ADME problems that exist for many drugs and drug candidates. One inherent problem is the rapid metabolism that causes a number of drugs, which otherwise would be highly effective in treating a disease, to be cleared too rapidly from the body. A possible solution to rapid drug clearance is frequent or high dosing to attain a sufficiently high plasma level of drug. This, however, introduces a number of potential treatment problems, such as poor patient compliance with the dosing regimen, side effects that become more acute with higher doses, and increased cost of treatment.

In some select cases, a metabolic inhibitor will be co-administered with an important drug that is rapidly cleared. Such is the case with the protease inhibitor class of drugs that are used to treat HIV infection. These drugs are typically co-dosed with ritonavir, an inhibitor of cytochrome P450 enzyme CYP3A4, the enzyme responsible for their metabolism. Ritonavir itself has side effects and it adds to the pill burden for HIV patients who must already take a combination of different drugs. Similarly, dextromethorphan which undergoes rapid CYP2D6 metabolism is being tested in combination with the CYP2D6 inhibitor quinidine for the treatment of pseudobulbar disease.

In general, combining drugs with cytochrome P450 inhibitors is not a satisfactory strategy for decreasing drug clearance. The inhibition of a CYP enzyme activity can affect the metabolism and clearance of other drugs metabolized by that same enzyme. This can cause those other drugs to accumulate in the body to toxic levels.

A potentially attractive strategy, if it works, for improving a drug's metabolic properties is deuterium modification. In this approach, one attempts to slow the CYP-mediated metabolism of a drug by replacing one or more hydrogen atoms with deuterium atoms. Deuterium is a safe, stable, non-radioactive isotope of hydrogen. Deuterium forms stronger bonds with carbon than hydrogen does. In select cases, the increased bond strength imparted by deuterium can positively impact the ADME properties of a drug, creating the potential for improved drug efficacy, safety, and tolerability. At the same time, because the size and shape of deuterium are essentially identical to hydrogen, replacement of hydrogen by deuterium would not be expected to affect the biochemical potency and selectivity of the drug as compared to the original chemical entity that contains only hydrogen.

Over the past 35 years, the effects of deuterium substitution on the rate of metabolism have been reported for a very small percentage of approved drugs (see, e.g., Blake, MI et al, J Pharm Sci, 1975, 64:367-91; Foster, AB, Adv Drug Res 1985, 14:1-40 ("Foster"); Kushner, DJ et al, Can J Physiol Pharmacol 1999, 79-88; Fisher, MB et al, Curr Opin Drug Discov Devel, 2006, 9:101-09 ("Fisher")). The results have been variable and unpredictable. For some compounds deuteration caused decreased metabolic clearance *in vivo.* For others, there was no change in metabolism. Still others demonstrated decreased metabolic clearance. The variability in deuterium effects has also led experts to question or dismiss deuterium modification as a viable drug design strategy for inhibiting adverse metabolism. (See Foster at p. 35 and Fisher at p. 101).

The effects of deuterium modification on a drug's metabolic properties are not predictable even when deuterium atoms are incorporated at known sites of metabolism. Only by actually preparing and testing a deuterated drug can one determine if and how the rate of metabolism will differ from that of its undeuterated counterpart. Many drugs have multiple sites where metabolism is possible. The site(s) where deuterium substitution is required and the extent of deuteration necessary to see an effect on metabolism, if any, will be different for each drug.

### SUMMARY OF THE INVENTION

This invention relates to novel compounds that are substituted xanthine derivatives and pharmaceutically acceptable salts thereof. For example, this invention relates to novel substituted xanthine derivatives that are structurally related to pentoxifylline. This invention also provides compositions comprising one or more compounds of this invention and a carrier and the disclosed compounds and compositions for use in methods of treating diseases and conditions for which pentoxifylline and related compounds are beneficial. Biochemical Pharmacology, 2007, 74, 639-646 discloses metabolites of pentoxifylline.

### BRIEF DESCRIPTION OF THE DRAWINGS

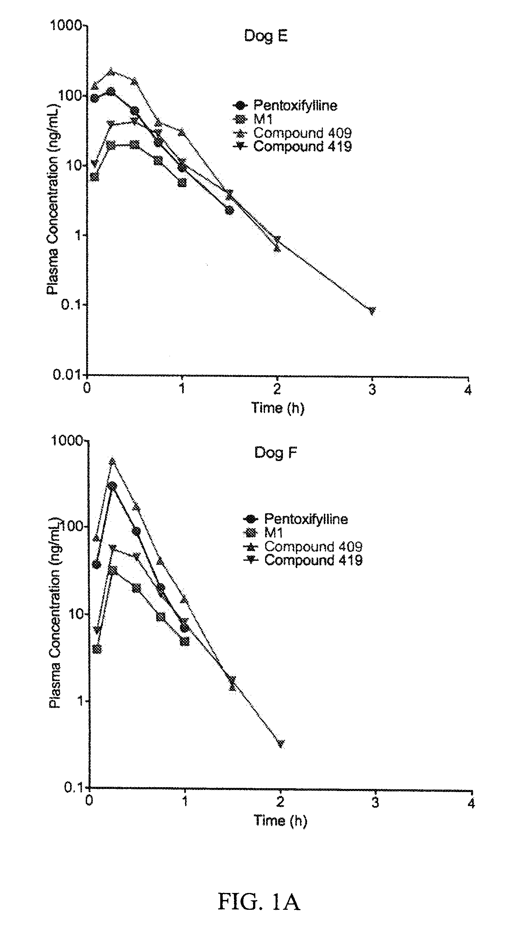

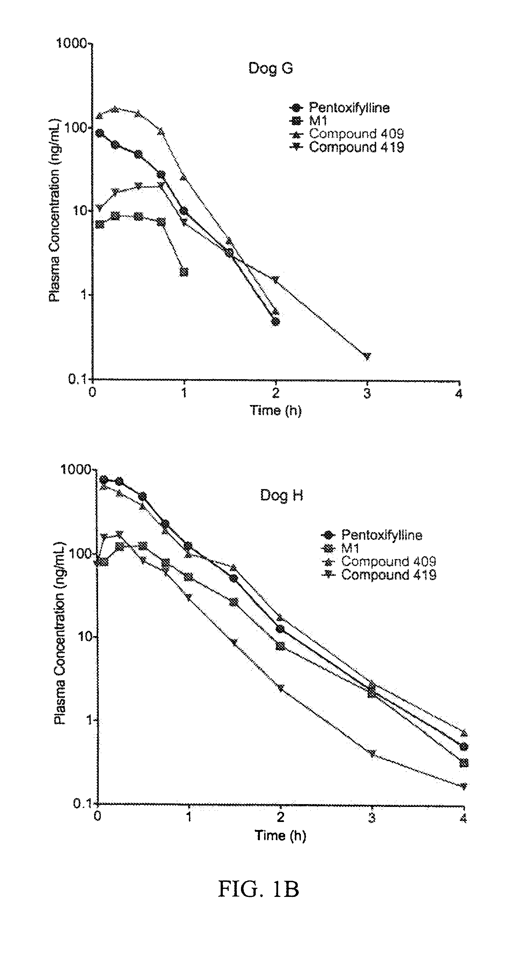

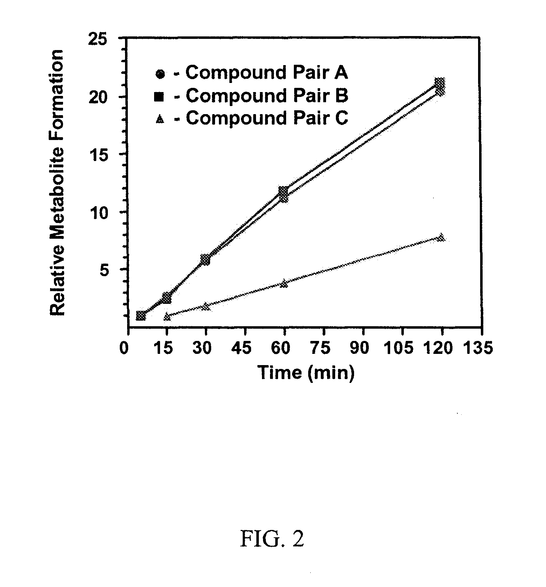

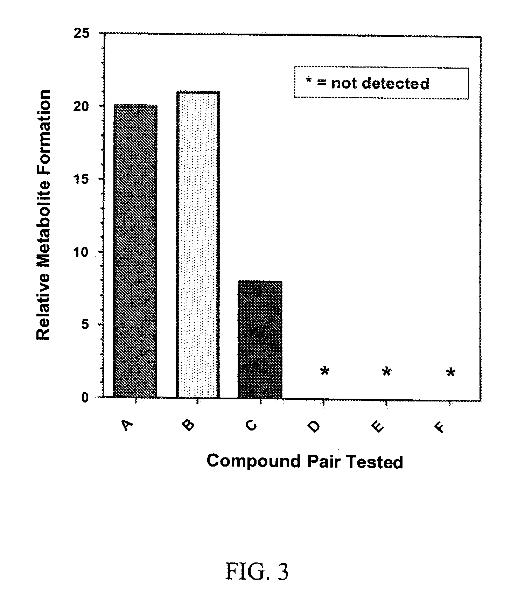

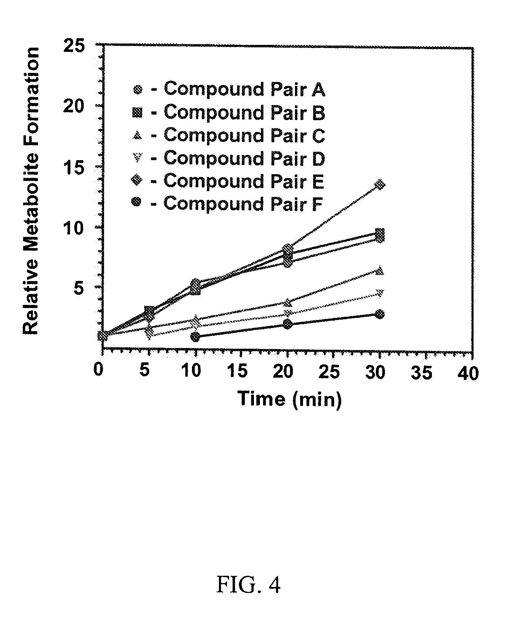

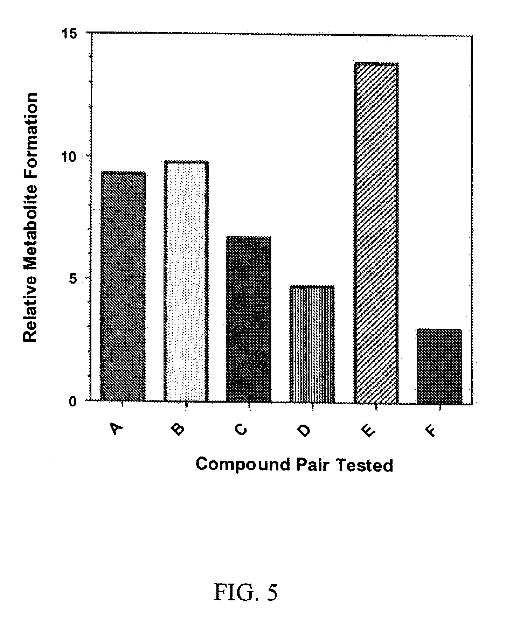

FIGS. 1A and 1B depict the serum levels of a compound described herein, pentoxifylline and certain of their respective metabolites in four individual dogs following oral administration of a combination of pentoxifylline and that compound of this invention. FIG. 2 depicts the time course of the production of the specific metabolites measured in FIG. 3 following incubation of various compounds described herein, pentoxifylline, (S)-M1 and (R)-M1 with rat whole blood. FIG. 3 depicts the relative amount of specific metabolites produced following incubation of various compounds described herein, pentoxifylline, (S)-M1 and (R)-M1 with rat whole blood. FIG. 4 depicts the time course of the production of the specific metabolites measured in FIG. 5 following incubation of various compounds of described herein, pentoxifylline, (S)-M1 and (R)-M1 with human liver microsomes. FIG. 5 depicts the relative amount of specific metabolites produced following incubation of various compounds described herein, pentoxifylline, (S)-M1 and (R)-M1 with human liver microsomes.

### DETAILED DESCRIPTION OF THE INVENTION

The terms "ameliorate" and "treat" are used interchangeably and include both therapeutic and prophylactic treatment. Both terms mean decrease, suppress, attenuate, diminish, arrest, or stabilize the development or progression of a disease (e.g., a disease or disorder delineated herein), lessen the severity of the disease or improve the symptoms associated with the disease.

"Disease" means any condition or disorder that damages or interferes with the normal function of a cell, tissue, or organ.

It will be recognized that some variation of natural isotopic abundance occurs in a synthesized compound depending upon the origin of chemical materials used in the synthesis. Thus, a preparation of pentoxifylline will inherently contain small amounts of deuterated isotopologues. The concentration of naturally abundant stable hydrogen and carbon isotopes, notwithstanding this variation, is small and immaterial as compared to the degree of stable isotopic substitution of compounds of this invention. See, for instance, Wada E et al., Seikagaku, 1994, 66: 15; Gannes LZ et al., Comp Biochem Physiol Mol Integr Physiol, 1998, 119: 725. In a compound of this invention, when a particular position is designated as having deuterium, it is understood that the abundance of deuterium at that position is substantially greater than the natural abundance of deuterium, which is 0.015%. A position designated as having deuterium typically has a minimum isotopic enrichment factor of at least 5000 (75% deuterium incorporation) at each atom designated as deuterium in said compound.

The term "isotopic enrichment factor" as used herein means the ratio between the isotopic abundance and the natural abundance of a specified isotope.

A compound of this invention has an isotopic enrichment factor for each designated deuterium atom of at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

In the compounds of this invention any atom not specifically designated as a particular isotope is meant to represent any stable isotope of that atom. Unless otherwise stated, when a position is designated specifically as "H" or "hydrogen", the position is understood to have hydrogen at its natural abundance isotopic composition. Also unless otherwise stated, when a position is designated specifically as "D" or "deuterium", the position is understood to have deuterium at an abundance that is at least 5000 times greater than the natural abundance of deuterium, which is 0.015% (i.e., at least 75% incorporation of deuterium).

The term "isotopologue" refers to a species that differs from a specific compound of this invention only in the isotopic composition thereof.

The term "compound," when referring to a compound of this invention, refers to a collection of molecules having an identical chemical structure, except that there may be isotopic variation among the constituent atoms of the molecules. Thus, it will be clear to those of skill in the art that a compound represented by a particular chemical structure containing indicated deuterium atoms, will also contain lesser amounts of isotopologues having hydrogen atoms at one or more of the designated deuterium positions in that structure. The relative amount of such isotopologues in a compound of this invention will depend upon a number of factors including the isotopic purity of deuterated reagents used to make the compound and the efficiency of incorporation of deuterium in the various synthesis steps used to prepare the compound. However, as set forth above, the relative amount of such isotopologues *in toto* will be less than 49.9% of the compound.

The invention also provides salts of the compounds of the invention. A salt of a compound of this invention is formed between an acid and a basic group of the compound, such as an amino functional group, or a base and an acidic group of the compound, such as a carboxyl functional group. According to another embodiment, the compound is a pharmaceutically acceptable acid addition salt.

The term "pharmaceutically acceptable," as used herein, refers to a component that is, within the scope of sound medical judgment, suitable for use in contact with the tissues of humans and other mammals without undue toxicity, irritation, allergic response and the like, and are commensurate with a reasonable benefit/risk ratio. A "pharmaceutically acceptable salt" means any non-toxic salt that, upon administration to a recipient, is capable of providing, either directly or indirectly, a compound of this invention. A "pharmaceutically acceptable counterion" is an ionic portion of a salt that is not toxic when released from the salt upon administration to a recipient.

Acids commonly employed to form pharmaceutically acceptable salts include inorganic acids such as hydrogen sulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2-sulfonate, mandelate and other salts. In one embodiment, pharmaceutically acceptable acid addition salts include those formed with mineral acids such as hydrochloric acid and hydrobromic acid, and especially those formed with organic acids such as maleic acid.

The invention also includes solvates and hydrates of the compound of the invention. As used herein, the term "hydrate" means a compound which further includes a stoichiometric or non-stoichiometric amount of water bound by non-covalent intermolecular forces. As used herein, the term "solvate" means a compound which further includes a stoichiometric or non-stoichiometric amount of solvent such as water, acetone, ethanol, methanol, dichloromethane, 2-propanol, or the like, bound by non-covalent intermolecular forces.

It is understood that the carbon atom that bears substituents H and OH in compound 121(*S*) is chiral (as H is different from OH). The chiral compounds of the present invention are present in the form of individual respective stereoisomers that are substantially free from another possible stereoisomer. The term "substantially free of other stereoisomers" as used herein means less than 25% of other stereoisomers, preferably less than 10% of other stereoisomers, more preferably less than 5% of other stereoisomers and most preferably less than 2% of other stereoisomers, or less than "X"% of other stereoisomers (wherein X is a number between 0 and 100, inclusive) are present. Methods of obtaining or synthesizing an individual enantiomer for a given compound are well known in the art and may be applied as practicable to final compounds or to starting material or intermediates.

Unless otherwise indicated, when a disclosed compound is named or depicted by a structure without specifying the stereochemistry and has one or more chiral centers, it is understood to represent all possible stereoisomers of the compound.

The term "stable compounds," as used herein, refers to compounds which possess stability sufficient to allow for their manufacture and which maintain the integrity of the compound for a sufficient period of time to be useful for the purposes detailed herein (e.g., formulation into therapeutic products, intermediates for use in production of therapeutic compounds, isolatable or storable intermediate compounds, treating a disease or condition responsive to therapeutic agents).

"D" refers to deuterium. "Stereoisomer" refers to both enantiomers and diastereomers. "Tert", "^{t}", and "t-" each refer to tertiary. "US" refers to the United States of America.

The term "optionally substituted with deuterium" means that one or more hydrogen atoms in the referenced moiety or compound may be replaced with a corresponding number of deuterium atoms.

Throughout this specification, a variable may be referred to generally (e.g.,"each R") or may be referred to specifically (e.g., R¹, R², etc.). Unless otherwise indicated, when a variable is referred to generally, it is meant to include all specific embodiments of that particular variable.

### THERAPEUTIC COMPOUNDS

The present invention provides in one aspect the following compound or a pharmaceutically acceptable salt thereof:

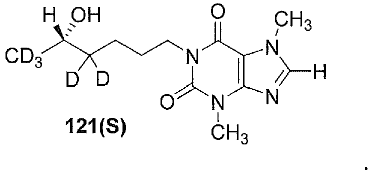

In another set of embodiments, any atom not designated as deuterium in any of the embodiments set forth above is present at its natural isotopic abundance.

The synthesis of compounds of this invention can be achieved by synthetic chemists of ordinary skill. Relevant procedures and intermediates are disclosed, for instance in Sidzhakova, D et al., Farmatsiya, (Sofia, Bulgaria) 1988, 38(4): 1-5; Davis, PJ et al., Xenobiotica, 1985, 15(12): 1001-10; Akgun, H et al., J Pharm Sci, 2001, 26(2): 67-71; German Patent publication DD 274334; Czech Patent Nos. CS 237719, CS201558; PCT patent publication WO9531450; and in Japanese Patent publication Nos. JP58150594, JP58134092, JP58038284, JP57200391, JP57098284, JP57085387, JP57062278, JP57080385, JP57056481, JP57024385, JP57011981, JP57024386, JP57024382, JP56077279, JP56032477, JP56007785, JP56010188, JP56010187, JP55122779, and JP55076876.

Such methods can be carried out utilizing corresponding deuterated and optionally, other isotope-containing reagents and/or intermediates to synthesize the compounds delineated herein, or invoking standard synthetic protocols known in the art for introducing isotopic atoms to a chemical structure.

### EXEMPLARY SYNTHESIS

Methods for synthesizing compounds of the invention are depicted in the following schemes.

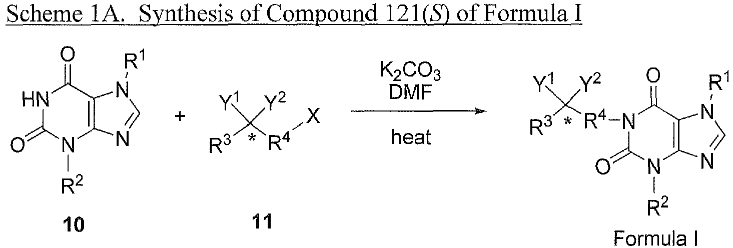

In Schemes 1A and 1B, R¹ is CH₃, R² is CH₃, Y² is hydrogen, R³ is CD₃, R⁴ is ^{†}CD₂(CH₂)₃ (wherein ^{†} represents the portion of the R⁴ moiety bound to C(Y¹)(Y²)), and Y¹ is OH.

As depicted in Scheme 1A, deuterated compound 10 is alkylated with deuterated intermediate 11 (wherein X is chloride, bromide or iodide) in the presence of potassium carbonate to afford compounds of Formula I. Alternatively, sodium hydroxide in aqueous methanol may be employed to afford compounds of Formula I according to the methods of US Patent 4289776.

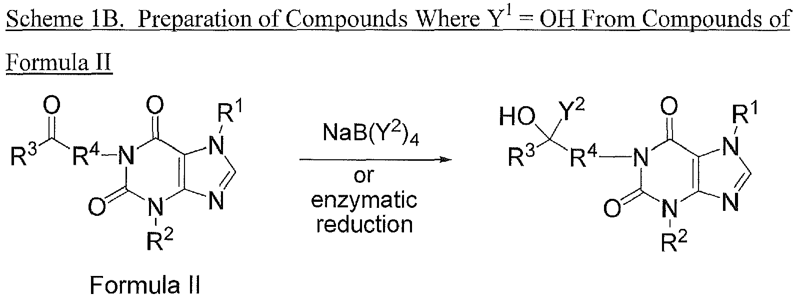

As depicted in Scheme 1 B, compounds of Formula II can be used to make compounds where Y¹ is OH. Thus, compounds of Formula II are reduced with either sodium borohydride or sodium borodeuteride (commercially available at 99 atom %D) according to the general method of European Patent publication 0330031 to form compounds wherein Y¹ is OH and Y² is hydrogen. The enantiomeric alcohol products may be separated, for example through the method of Nicklasson, M et al., Chirality, 2002, 14(8): 643-652. In an alternate method, enzymatic reduction affords an enantiomerically-enriched alcohol product using the methods disclosed in Pekala, E et al., Acta Poloniae Pharmaceutica, 2007, 64(2): 109-113, or in Pekala, E et al., Biotech J, 2007, 2(4): 492-496.

Stereoselective preparation of compounds where C(Y¹)(Y²) is C(H)OH from corresponding compounds where C(Y¹)(Y²) is C=O may be carried out in the presence of a ketoreductase or carbonyl reductase. Compounds of the invention where C(Y¹)(Y²) is C(H)OH may be prepared stereoselectively from corresponding compounds where C(Y¹)(Y²) is C=O by treating with a hydride source in the presence of a ketoreductase or carbonyl reductase at an appropriate pH with an enantiomeric excess of at least 80%. The ketoreductase or carbonyl reductase that favors formation of a compound wherein the stereochemistry at the C(H)OH or group is (S) may be, for example, any one of ALMAC Carbonyl Reductases CRED A131, CRED A801, CRED A901, CRED A251, or CRED A271 (each commercially available from ALMAC Group Ltd, Craigavon, England), any one of CODEXIS Ketoreductases KRED-119, KRED-137, KRED-148, KRED-169, KRED-174, KRED-NADH 101, KRED-NADH 102, KRED-NADH112, or KRED-NADH 126 (each commercially available from Codexis Inc., Redwood City, CA), or SYNCORE Ketoreductases ES-KRED-121, ES-KRED-128, ES-KRED-130, ES-KRED-142, ES-KRED-175, ES-KRED-169, or ES-KRED-171 (each commercially available from Syncore Labs, Shanghai, China). In one aspect of, the enzyme is selected from CRED A131, CRED A251, KRED-NADH 101, KRED-NADH 102, KRED-NADH 112, KRED-NADH 126, ES-KRED-121, ES-KRED-128, ES-KRED-130, ES-KRED-142, ES-KRED-169, or ES-KRED-171. In a more specific aspect, the enzyme is selected from CRED A131, CRED A251, and KRED-NADH 101.

The amount of ketoreductase or carbonyl reductase used in the reaction ranges from 0.05 wt% to 10 wt% as a percentage of the weight of the substrate, such as 0.5 wt% to 5 wt%. In one embodiment, the amount of enzyme is between 1.0 wt% and 2.0 wt%. In a more specific aspect, the amount of enzyme is about 1.0 wt%. The hydride source is a compound or mixture that is capable of providing a hydride anion or a synthon of a hydride anion. The deuteride source is a compound or mixture that is capable of providing a deuteride anion or a synthon of a deuteride anion. A hydride or deuteride source comprises a catalytic co-factor and optionally, a co-factor regeneration system. A catalytic co-factor used with the ketone reductase or carbonyl reductase in the process of this invention is selected from NAD, NADP, NADH, NADPH, NAD²H and NADP²H. The choice of co-factor may be based upon (a) the presence or absence of a co-factor regeneration system; (b) the requirement for a hydride versus a deuteride source; and (c) an appropriate pH to perform the method according to the present invention means buffer conditions that maintain the pH at between 6.0 and 7.5 throughout the reaction. In one embodiment, the pH of the reaction was maintained at between 6.5 and 7.3. In another embodiment, the pH of the reaction was maintained between 6.0 and 7.0. Typically dropwise addition of KOH is used to maintain the desired pH because the enzymatic reaction generates acid. In one aspect, the pH of the reaction is maintained between 6.90 and 7.05. The process may be performed at a temperature of about 20° C to 37° C. In one aspect of this embodiment, the temperature is about 29° C to 32° C. The process may be performed over a time period of about 12 hours to about 24 hours. In one embodiment, the time period is about 24 hours to about 40 hours. In one embodiment, the time period is about 40 hours to about 72 hours. In one embodiment, the time period is a time period sufficient for less than about 5% of the initial amount of the compound wherein C(Y¹)(Y²) is C=O to be present.

### Synthesis of Compound 10

Referring to Scheme 1A, compounds that can be used as compound **10** to make compounds of Formula I are known and include, but are not limited to, the following: theobromine (wherein R¹ and R² are CH₃) which is commercially available. Isotopologues of **10** wherein: (a) R¹ is -CD₃ and R² is -CH₃; (b) R¹ is-CH₃ and R² is -CD₃; and (c) R¹ and R² are -CD₃ are all known. *See* Benchekroun, Y et al., J Chromatogr B, 1977, 688: 245; Ribon, B et al., Coll INSERM, 1988, 164: 268; and Horning, MG et al., Proc Int Conf Stable Isot 2nd, 1976, 41-54.

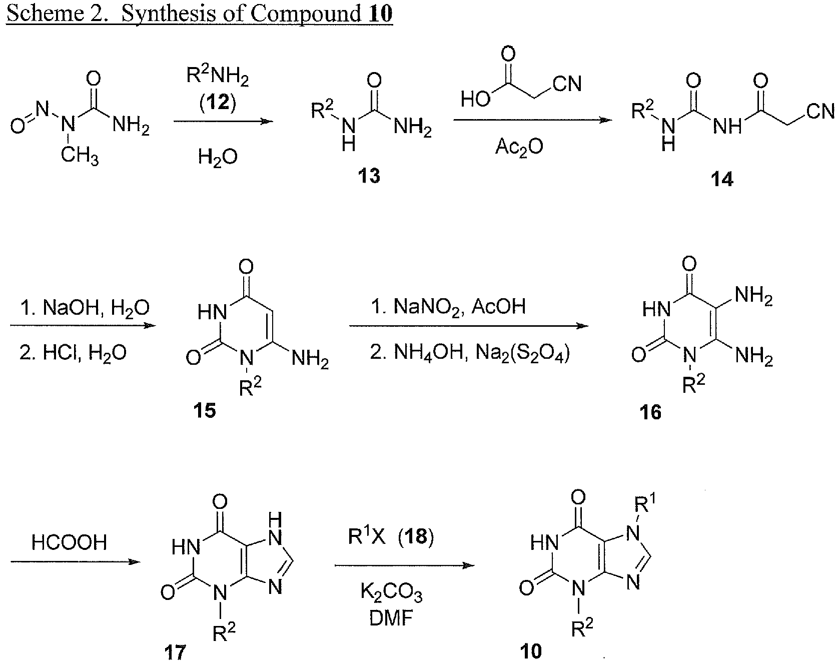

A synthesis of compound **10** is depicted in Scheme 2 (in which R¹ is CH₃ and R² is CH₃) starting with commercially-available N-nitroso-N-methylurea. Treatment with appropriately deuterated amine **12** in water affords N-alkylurea **13** following the methods of Boivin, JL et al., Canadian Journal of Chemistry, 1951, 29: 478-81. Urea **13** may be treated with 2-cyanoacetic acid and acetic anhydride to provide cyanoacetamide derivative **14,** which is treated first with aqueous NaOH and then with aqueous HCl to provide cyclized pyrimidinedione **15** according to the methods of Dubey, PK et al., Indian Journal of Heterocyclic Chemistry, 2005, 14(4): 301-306. Alternatively, cyanoacetamide **14** may be treated with trimethylsilylchloride and hexamethyldisilazane to afford the cyclized product **15** via the methods of Fulle, F et al., Heterocycles, 2000, 53(2): 347-352.

Following the methods of Merlos, M et al., European Journal of Medicinal Chemistry, 1990, 25(8): 653-8, treatment of pyrimidinedione **15** with sodium nitrite in acetic acid, and then by ammonium hydroxide and sodium dithionite, yields compound **16,** which is treated with formic acid to provide purine derivative **17.** Following the methods disclosed by Rybar, A et al., in Czech patent application CS 263595B1, alkylation of **17** with appropriately deuterated electrophile **18** (X is chloro, bromo, or iodo) in the presence of potassium carbonate and optionally in the presence of additives such as NaBr, KBr, NaI, KI, or iodine, affords compound **10.**

Referring to Scheme 2, useful deuterated amine reagents **12** include, but are not limited to, commercially-available compounds such as n-propyl-d₇-amine, or known compounds such as 1-propan-1,1-d₂-amine (Moritz, F et al., Organic Mass Spectrometry, 1993, 28(3): 207-15). Useful deuterated urea reagents **13** may include, but are not limited to, commercially-available compounds such as N-methyl-d₃-urea 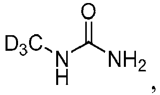 or methylurea-d₆

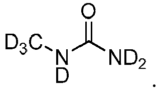

Useful deuterated electrophiles **18** may include, but are not limited to, commercially-available compounds such as iodomethane-d₃, or bromomethane-d₃, or 1-bromopropane-d₇, or 1-bromopropane-1,1-d₂, or known compounds such as (chloromethoxy-d₂)-ethane (Williams, AG, WO 2002059070A1), or bromomethoxymethane-d₂ (Van der Veken, BJ et al., Journal of Raman Spectroscopy, 1992, 23(4): 205-23, or (bromomethoxy-d₂)-methane-d₃ (Van der Veken, BJ et al., Journal of Raman Spectroscopy, 1992, 23(4): 205-23. The commercially available deuterated intermediates **12, 13** and **18** mentioned above are available having an isotopic purity of at least 98 atom % D.

### Synthesis of Intermediate 11a-d₅ (cf. Scheme 1A)

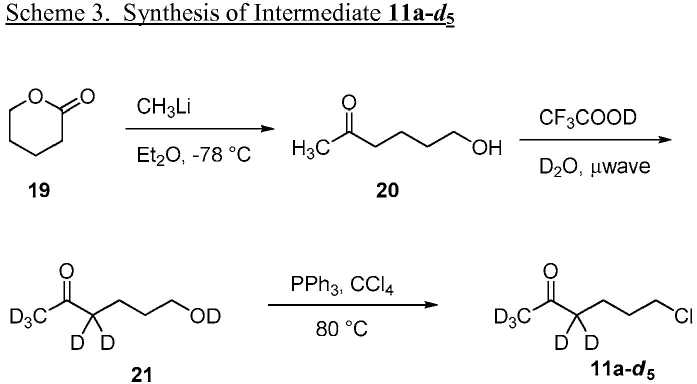

An approach to the preparation of compound **11a-*d*₅** (cf. Scheme 1A) (wherein R³ is CD₃; R⁴ is ^{†}-CD₂(CH₂)₃-, and Y¹ and Y² are taken together to form =O), is depicted in Scheme 3. Thus, methyllithium is added to commercially-available delta-valerolactone **19** according to the procedure of Zhang, Q et al., Tetrahedron, 2006, 62(50): 11627-11634 to afford ketone **20.** Treatment of **20** with TFA-*d₁* (99 atom %D) in D₂O (99 atom %D) under microwave conditions provides deuterated ketone **21** according to the general method of Fodor-Csorba K, Tet Lett, 2002, 43: 3789-3792. The alcohol moiety in **21** is converted to the chloride upon treatment with triphenylphosphine and carbon tetrachloride to yield chloride **11a-*d*₅** following the general procedures of Clement, J-L, Org Biomol Chem, 2003, 1: 1591-1597.

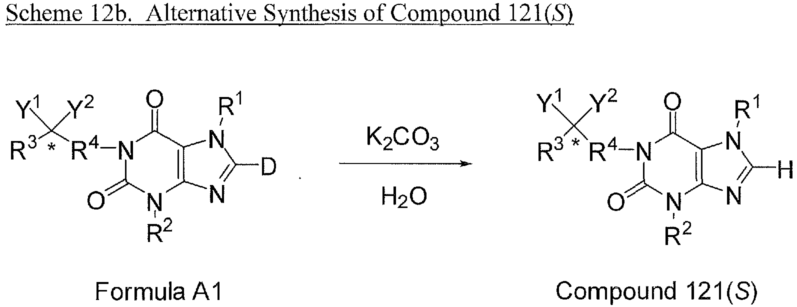

An alternative synthesis of the compound 121(*S*) is depicted in Scheme 12b. Thus, compounds of general Formula A1 are treated with potassium carbonate in water to effect a deuterium-to-hydrogen exchange, which affords the compound 121(*S*). In the method of Scheme 12b, R¹ and R² are both CH₃, R³ is CD₃, R⁴ is ⁺CD₂(CH₂)₃ (wherein ⁺ represents the portion of the R⁴ moiety bound to C(Y¹)(Y²)), Y¹ is OH and Y² is hydrogen.

A number of novel intermediates can be used to prepare compound 121(*S*).

The specific approaches and compounds shown above are not intended to be limiting. The chemical structures in the schemes herein depict variables that are hereby defined commensurately with chemical group definitions (moieties, atoms, etc.) of the corresponding position in the compound formulae herein, whether identified by the same variable name (i.e., R¹, R², etc.) or not. The suitability of a chemical group in a compound structure for use in the synthesis of another compound is within the knowledge of one of ordinary skill in the art. Additional methods of synthesizing compounds of this invention and their synthetic precursors, including those within routes not explicitly shown in schemes herein, are within the means of chemists of ordinary skill in the art. Synthetic chemistry transformations and protecting group methodologies (protection and deprotection) useful in synthesizing the applicable compounds are known in the art and include, for example, those described in Larock R, Comprehensive Organic Transformations, VCH Publishers (1989); Greene TW et al., Protective Groups in Organic Synthesis, 3rd Ed., John Wiley and Sons (1999); Fieser L et al., Fieser and Fieser's Reagents for Organic Synthesis, John Wiley and Sons (1994); and Paquette L, ed., Encyclopedia of Reagents for Organic Synthesis, John Wiley and Sons (1995) and subsequent editions thereof.

Combinations of substituents and variables envisioned by this invention are only those that result in the formation of stable compounds.

### COMPOSITIONS

The invention also provides pyrogen-free compositions comprising an effective amount of a compound of this invention or pharmaceutically acceptable salts thereof; and an acceptable carrier. Preferably, a composition of this invention is formulated for pharmaceutical use ("a pharmaceutical composition"), wherein the carrier is a pharmaceutically acceptable carrier. The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions of this invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, microcrystalline cellulose, cellulose-based substances, polyethylene glycol, sodium carboxymethylcellulose, polyacrylates, waxes, polyethylene-polyoxypropylene-block polymers, polyethylene glycol and wool fat.

If required, the solubility and bioavailability of the compounds of the present invention in pharmaceutical compositions may be enhanced by methods well-known in the art. One method includes the use of lipid excipients in the formulation. See "Oral Lipid-Based Formulations: Enhancing the Bioavailability of Poorly WaterSoluble Drugs (Drugs and the Pharmaceutical Sciences)," David J. Hauss, ed. Informa Healthcare, 2007; and "Role of Lipid Excipients in Modifying Oral and Parenteral Drug Delivery: Basic Principles and Biological Examples," Kishor M. Wasan, ed. Wiley-Interscience, 2006.

Another known method of enhancing bioavailability is the use of an amorphous form of a compound of this invention optionally formulated with a poloxamer, such as LUTROL™ and PLURONIC™ (BASF Corporation), or block copolymers of ethylene oxide and propylene oxide. See United States patent 7,014,866; and United States patent publications 20060094744 and 20060079502.

The pharmaceutical compositions of the invention include those suitable for oral, rectal, nasal, topical (including buccal and sublingual), vaginal or parenteral (including subcutaneous, intramuscular, intravenous and intradermal) administration. In certain embodiments, the compound of the formulae herein is administered transdermally (e.g., using a transdermal patch or iontophoretic techniques). Other formulations may conveniently be presented in unit dosage form, e.g., tablets, sustained release capsules, and in liposomes, and may be prepared by any methods well known in the art of pharmacy. See, for example, Remington's Pharmaceutical Sciences, Mack Publishing Company, Philadelphia, PA (17th ed. 1985).

Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product.

In certain embodiments, the compound is administered orally. Compositions of the present invention suitable for oral administration may be presented as discrete units such as capsules, sachets, or tablets each containing a predetermined amount of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption.

In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, immediately prior to use. Extemporaneous injection solutions and suspensions may be prepared from sterile powders, granules and tablets.

Such injection solutions may be in the form, for example, of a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of this invention may be administered in the form of suppositories for rectal administration. These compositions can be prepared by mixing a compound of this invention with a suitable non-irritating excipient which is solid at room temperature but liquid at the rectal temperature and therefore will melt in the rectum to release the active components. Such materials include, but are not limited to, cocoa butter, beeswax and polyethylene glycols.

The pharmaceutical compositions of this invention may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, e.g.: Rabinowitz, JD and Zaffaroni, AC, US Patent 6,803,031, assigned to Alexza Molecular Delivery Corporation.

Topical administration of the pharmaceutical compositions of this invention is especially useful when the desired treatment involves areas or organs readily accessible by topical application. For topical application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene polyoxypropylene compound, emulsifying wax, and water. Alternatively, the pharmaceutical composition can be formulated with a suitable lotion or cream containing the active compound suspended or dissolved in a carrier. Suitable carriers include, but are not limited to, mineral oil, sorbitan monostearate, polysorbate 60, cetyl esters wax, cetearyl alcohol, 2-octyldodecanol, benzyl alcohol, and water. The pharmaceutical compositions of this invention may also be topically applied to the lower intestinal tract by rectal suppository formulation or in a suitable enema formulation. Topically-transdermal patches and iontophoretic administration are also included in this invention.

Application of the subject therapeutics may be local, so as to be administered at the site of interest. Various techniques can be used for providing the subject compositions at the site of interest, such as injection, use of catheters, trocars, projectiles, pluronic gel, stents, sustained drug release polymers or other device which provides for internal access.

Thus, according to yet another embodiment, the compounds of this invention may be incorporated into compositions for coating an implantable medical device, such as prostheses, artificial valves, vascular grafts, stents, or catheters. Suitable coatings and the general preparation of coated implantable devices are known in the art and are exemplified in US Patents 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition. Coatings for invasive devices are to be included within the definition of pharmaceutically acceptable carrier, adjuvant or vehicle, as those terms are used herein.

According to another embodiment, the invention provides a method of coating an implantable medical device comprising the step of contacting said device with the coating composition described above. It will be obvious to those skilled in the art that the coating of the device will occur prior to implantation into a mammal.

According to another embodiment, the invention provides a method of impregnating an implantable drug release device comprising the step of contacting said drug release device with a compound or composition of this invention. Implantable drug release devices include, but are not limited to, biodegradable polymer capsules or bullets, non-degradable, diffusible polymer capsules and biodegradable polymer wafers.

According to another embodiment, the invention provides an implantable medical device coated with a compound or a composition comprising a compound of this invention, such that said compound is therapeutically active.

According to another embodiment, the invention provides an implantable drug release device impregnated with or containing a compound or a composition comprising a compound of this invention, such that said compound is released from said device and is therapeutically active.

Where an organ or tissue is accessible because of removal from the patient, such organ or tissue may be bathed in a medium containing a composition of this invention, a composition of this invention may be painted onto the organ, or a composition of this invention may be applied in any other convenient way. In another embodiment, the compound of the inveniton comprises between 28 and 68% (w/w) of the composition. In this embodiment, magnesium stearate and microcrystalline cellulose comprise about 2% (w/w) of the composition.

In another embodiment, a composition of this invention further comprises a second therapeutic agent. The second therapeutic agent may be selected from any compound or therapeutic agent known to have or that demonstrates advantageous properties when administered with a compound having the same mechanism of action as pentoxifylline. Such agents include those indicated as being useful in combination with pentoxifylline, including but not limited to, those described in WO 1997019686, EP 0640342, WO 2003013568, WO 2001032156, WO 2006035418, and WO 1996005838.

Preferably, the second therapeutic agent is an agent useful in the treatment or prevention of a disease or condition selected from peripheral obstructive vascular disease; glomerulonephritis; nephrotic syndrome; nonalcoholic steatohepatitis; Leishmaniasis; cirrhosis; liver failure; Duchenne's muscular dystrophy; late radiation induced injuries; radiation induced lymphedema; radiation-associated necrosis; alcoholic hepatitis; radiation-associated fibrosis; necrotizing enterocolitis in premature neonates; diabetic nephropathy, hypertension-induced renal failure, and other chronic kidney disease; Focal Segmental Glomerulosclerosis; pulmonary sarcoidosis; recurrent aphthous stomatitis; chronic breast pain in breast cancer patients; brain and central nervous system tumors; malnutrition-inflammation-cachexia syndrome; interleukin-1 mediated disease; graft versus host reaction and other allograft reactions; diet-induced fatty liver conditions, atheromatous lesions, fatty liver degeneration and other diet-induced high fat or alcohol-induced tissue-degenerative conditions; human immunodeficiency virus type 1 (HIV-1) and other human retroviral infections; multiple sclerosis; cancer; fibroproliferative diseases; fungal infection; drug-induced nephrotoxicity; collagenous colitis and other diseases and/or conditions characterized by elevated levels of platelet derived growth factor (PDGF) or other inflammatory cytokines; endometriosis; optic neuropathy and CNS impairments associated with acquired immunodeficiency syndrome (AIDS), immune disorder diseases, or multiple sclerosis; autoimmune disease; upper respiratory viral infection; depression; urinary incontinence; irritable bowel syndrome; septic shock; Alzheimers Dementia; neuropathic pain; dysuria; retinal or optic nerve damage; peptic ulcer; insulin-dependent diabetes; non-insulin-dependent diabetes; diabetic nephropathy; metabolic syndrome; obesity; insulin resistance; dyslipidemia; pathological glucose tolerance; hypertension; hyperlipidemia; hyperuricemia; gout; hypercoagulability; and inflammation or injury associated with neutrophil chemotaxis and/or degranulation. The compounds of this invention can also be used to control intraocular pressure or to stabilize auto-regulation of cerebral blood flow in subjects who require such control as determined by medical examination.

In one embodiment, the second therapeutic agent is selected from α-tocopherol and hydroxyurea.

In another embodiment, the second therapeutic agent is useful in the treatment of diabetes or an associated disorder, and is selected from insulin or insulin analogues, glucagon-like-peptide-1 (GLP-1) receptor agonists, sulfonylurea agents, biguanide agents, alpha-glucosidase inhibitors, PPAR agonists, meglitinide agents, dipeptidyl-peptidase (DPP) IV inhibitors, other phosphodiesterase (PDE1, PDE5, PDE9, PDE10 or PDE1) inhibitors, amylin agonists, CoEnzyme A inhibitors, and antiobesity agents.

Specific examples of insulin include, but are not limited to Humulin® (human insulin, rDNA origin), Novolin® (human insulin, rDNA origin), Velosulin® BR (human buffered regular insulin, rDNA origin), Exubera® (human insulin, inhaled), and other forms of inhaled insulin, for instance, as delivered by Mannkind's "Technosphere Insulin System".

Specific examples of insulin analogues include, but are not limited to, novarapid, insulin detemir, insulin lispro, insulin glargine, insulin zinc suspension and Lys-Pro insulin.

Specific examples of Glucagon-Like-Peptide-1 receptor agonists include, but are not limited to BIM-51077 (CAS-No. 275371-94-3), EXENATIDE (CAS-No. 141758-74-9), CJC-1131 (CAS-No. 532951 -64-7), LIRAGLUTIDE (CAS-No. 20656-20-2) and ZP-10 (CAS-No. 320367-13-3).

Specific examples of sulfonylurea agents include, but are not limited to, TOLBUTAMIDE (CAS- No. 000064-77-7), TOLAZAMIDE (CAS-No. 001156-19-0), GLIPIZIDE (CAS-No. 029094-61-9), CARBUTAMIDE (CAS-No. 000339-43-5), GLISOXEPIDE (CAS-No. 025046-79-1), GLISENTIDE (CAS-No. 032797-92-5), GLIBORNURIDE (CAS-No. 026944-48-9), GLIBENCLAMIDE (CAS-NO. 010238-21 -8), GLIQUIDONE (CAS-No. 033342-05-1), GLIMEPIRIDE (CAS-No. 093479-97-1) and GLICLAZIDE (CAS-No. 021187-98-4).

A specific example of a biguanide agent includes, but is not limited to METFORMIN (CAS-No. 000657-24-9).

Specific examples of alpha-glucosidase-inhibitors include, but are not limited to ACARBOSE (Cas-No. 056180-94-0), MIGLITOL (CAS-No. 072432-03-2) and VOGLIBOSE (CAS-No. 083480-29-9).

Specific examples of PPAR-agonists include, but are not limited to MURAGLITAZAR (CAS-No. 331741 -94-7), ROSIGLITAZONE (CAS-NO. 122320-73-4), PIOGLITAZONE (CAS-No.111025-46-8), RAGAGLITAZAR (CAS-NO. 222834-30-2), FARGLITAZAR (CAS-No. 196808-45-4), TESAGLITAZAR (CAS- No. 251565-85-2), NAVEGLITAZAR (CAS-No. 476436-68-7), NETOGLITAZONE (CAS-NO. 161600-01 -7), RIVOGLITAZONE (CAS-NO. 185428-18-6), K-1 11 (CAS-No. 221564-97-2), GW-677954 (CAS-No. 622402-24-8), FK-614 (CAS-No 193012-35-0) and (-)-Halofenate (CAS-No. 024136-23-0). Preferred PPAR- agonists are ROSGLITAZONE and PIOGLITAZONE.

Specific examples of meglitinide agents include, but are not limited to REPAGLINIDE (CAS-No. 135062-02-1), NATEGLINIDE (CAS-No. 105816-04-4) and MITIGLINIDE (CAS-No. 145375-43-5).

Specific examples of DPP IV inhibitors include, but are not limited to SITAGLIPTIN (CAS-No. 486460-32-6), SAXAGLIPTIN (CAS-No. 361442-04-8), VILDAGLIPTIN (CAS-No. 274901 -16-5), DENAGLIPTIN (CAS-No. 483369-58-0), P32/98 (CAS-No. 251572-70-0) and NVP-DPP-728 (CAS-No. 247016-69-9).

Specific examples of PDE5 inhibitors include, but are not limited to SILDENAFIL (CAS-No. 139755-83-2), VARDENAFIL (CAS-No. 224785-90-4) and TADALAFIL (CAS-No. 171596-29-5). Examples of PDE1, PDE9, PDE10 or PDE11 inhibitors which may be usefully employed according to the present invention can be found, for example, in US20020160939, WO2003037432, US2004220186, WO2005/003129, WO2005012485, WO2005120514 and WO03077949.

A specific example of an amylin agonist includes, but is not limited to PRAMLINITIDE (CAS-No. 151126-32-8).

A specific example of a Coenzyme A inhibitor includes, but is not limited to ETOMOXIR (CAS- No. 082258-36-4).

Specific examples of anti-obesity drugs include, but are not limited to HMR-1426 (CAS-No. 262376-75-0), CETILISTAT (CAS-No. 282526-98-1) and SIBUTRAMINE (CAS-No. 106650-56-0).

In another embodiment, the invention provides separate dosage forms of a compound of this invention and one or more of any of the above-described second therapeutic agents, wherein the compound and second therapeutic agent are associated with one another. The term "associated with one another" as used herein means that the separate dosage forms are packaged together or otherwise attached to one another such that it is readily apparent that the separate dosage forms are intended to be sold and administered together (within less than 24 hours of one another, consecutively or simultaneously).

In the pharmaceutical compositions of the invention, the compound of the present invention is present in an effective amount. As used herein, the term "effective amount" refers to an amount which, when administered in a proper dosing regimen, is sufficient to treat (therapeutically or prophylactically) the target disorder. For example, and effective amount is sufficient to reduce or ameliorate the severity, duration or progression of the disorder being treated, prevent the advancement of the disorder being treated, cause the regression of the disorder being treated, or enhance or improve the prophylactic or therapeutic effect(s) of another therapy.

The interrelationship of dosages for animals and humans (based on milligrams per meter squared of body surface) is described in Freireich et al., Cancer Chemother. Rep, 1966, 50: 219. Body surface area may be determined approximately from height and weight of the patient. See, e.g., Scientific Tables, Geigy Pharmaceuticals, Ardsley, N.Y., 1970, 537.

In one embodiment, an effective amount of a compound of this invention is in the range of 20 mg to 2000 mg per treatment. In more specific embodiments the amount is in the range of 40 mg to 1000 mg, or in the range of 100 mg to 800 mg, or more specifically in the range of 200 mg to 400 mg per treatment. Treatment typically is administered from one to three times daily.

Effective doses will also vary, as recognized by those skilled in the art, depending on the diseases treated, the severity of the disease, the route of administration, the sex, age and general health condition of the patient, excipient usage, the possibility of co-usage with other therapeutic treatments such as use of other agents and the judgment of the treating physician. For example, guidance for selecting an effective dose can be determined by reference to the prescribing information for pentoxifylline.

For pharmaceutical compositions that comprise a second therapeutic agent, an effective amount of the second therapeutic agent is between about 20% and 100% of the dosage normally utilized in a monotherapy regime using just that agent. Preferably, an effective amount is between about 70% and 100% of the normal monotherapeutic dose. The normal monotherapeutic dosages of these second therapeutic agents are well known in the *art. See,* e.g., Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), each of which references are incorporated herein by reference in their entirety.

It is expected that some of the second therapeutic agents referenced above will act synergistically with the compounds of this invention. When this occurs, it will allow the effective dosage of the second therapeutic agent and/or the compound of this invention to be reduced from that required in a monotherapy. This has the advantage of minimizing toxic side effects of either the second therapeutic agent of a compound of this invention, synergistic improvements in efficacy, improved ease of administration or use and/or reduced overall expense of compound preparation or formulation.

### MEDICAL USES

A method of inhibiting the activity of phosphodiesterase (PDE) in a cell, comprising contacting a cell with compound 121(*S*) is described herein.

In addition to its PDE inhibitory activity, pentoxifylline is known to suppress the production of a number of other biological agents such as interleukin-1 (IL-1), IL-6, IL-12, TNF-alpha, fibrinogen, and various growth factors. Accordingly, a method of suppressing the production of interleukin-1 (IL-1), IL-6, IL-12, TNF-alpha, fibrinogen, and various growth factors in a cell, comprising contacting a cell with compound 121(*S*) is described herein.

A method of treating a disease in a patient in need thereof that is beneficially treated by pentoxifylline comprising the step of administering to said patient an effective amount of compound of 121(*S*), or a pharmaceutical composition comprising compound 121(*S*), and a pharmaceutically acceptable carrier is described herein.

Such diseases are well known in the art and are disclosed in, but not limited to the following patents and published applications: WO 1988004928, EP 0493682, US 5112827, EP 0484785, WO 1997019686, WO 2003013568, WO 2001032156, WO 1992007566, WO 1998055110, WO 2005023193, US 4975432, WO 1993018770, EP 0490181, and WO 1996005836.

In one embodiment, the invention provides compound of 121(*S*), for use in treating a disease selected from the group consisting of peripheral obstructive vascular disease; glomerulonephritis; nephrotic syndrome; nonalcoholic steatohepatitis; Leishmaniasis; cirrhosis; liver failure; Duchenne's muscular dystrophy; late radiation induced injuries; radiation induced lymphedema; radiation-associated necrosis; alcoholic hepatitis; radiation-associated fibrosis; necrotizing enterocolitis in premature neonates; diabetic nephropathy, hypertension-induced renal failure, and other chronic kidney disease; Focal Segmental Glomerulosclerosis; pulmonary sarcoidosis; recurrent aphthous stomatitis; chronic breast pain in breast cancer patients; brain and central nervous system tumors; malnutrition-inflammation-cachexia syndrome; interleukin-1 mediated disease; graft versus host reaction and other allograft reactions; diet-induced fatty liver conditions, atheromatous lesions, fatty liver degeneration and other diet-induced high fat or alcohol-induced tissue-degenerative conditions; human immunodeficiency virus type 1 (HIV-1) and other human retroviral infections; multiple sclerosis; cancer; fibroproliferative diseases; fungal infection; drug-induced nephrotoxicity; collagenous colitis and other diseases and/or conditions characterized by elevated levels of platelet derived growth factor (PDGF) or other inflammatory cytokines; endometriosis; optic neuropathy and CNS impairments associated with acquired immunodeficiency syndrome (AIDS), immune disorder diseases, or multiple sclerosis; autoimmune disease; upper respiratory viral infection; depression; urinary incontinence; irritable bowel syndrome; septic shock; Alzheimers Dementia; neuropathic pain; dysuria; retinal or optic nerve damage; peptic ulcer; insulin-dependent diabetes; non-insulin-dependent diabetes; diabetic nephropathy; metabolic syndrome; obesity; insulin resistance; dyslipidemia; pathological glucose tolerance; hypertension; hyperlipidemia; hyperuricemia; gout; hypercoagulability; acute alcoholic hepatitis; olfaction disorders; patent ductus arteriosus; and inflammation or injury associated with neutrophil chemotaxis and/or degranulation.

The compound 121(*S*) can also be used to control intraocular pressure or to stabilize auto-regulation of cerebral blood flow in subjects who require such control as determined by medical examination.

In one embodiment, the invention provides compound 121(*S*), for use in treating a disease or condition in a patient in need thereof selected from intermittent claudication on the basis of chronic occlusive arterial disease of the limbs and other peripheral obstructive vascular diseases; glomerulonephritis; Focal Segmental Glomerulosclerosis; nephrotic syndrome; nonalcoholic steatohepatitis; Leishmaniasis; cirrhosis; liver failure; Duchenne's muscular dystrophy; late radiation induced injuries; radiation induced lymphedema; alcoholic hepatitis; radiation-induced fibrosis; necrotizing enterocolitis in premature neonates; diabetic nephropathy, hypertension-induced renal failure and other chronic kidney diseases; pulmonary sarcoidosis; recurrent aphthous stomatitis; chronic breast pain in breast cancer patients; brain and central nervous system tumors; obesity; acute alcoholic hepatitis; olfaction disorders; endometriosis-associated infertility; malnutrition-inflammation-cachexia syndrome; and patent ductus arteriosus.

In one embodiment, the invention provides compound 121(*S*), for use in treating diabetic nephropathy, hypertensive nephropathy or intermittent claudication on the basis of chronic occlusive arterial disease of the limbs. In one specific aspect of this embodiment, the compound of this invention is used to treat diabetic nephropathy.

In one embodiment, the invention provides compound 121(*S*), for use in treating a disease or condition in a patient in need thereof selected from intermittent claudication on the basis of chronic occlusive arterial disease of the limbs.

In one embodiment, the invention provides compound 121(*S*), for use in treating chronic kidney disease. The chronic kidney disease may be selected from glomerulonephritis, focal segmental glomerulosclerosis, nephrotic syndrome, reflux uropathy, or polycystic kidney disease.

In one embodiment, the invention provides compound 121(*S*), for use in treating chronic disease of the liver. The chronic disease of the liver may be selected from nonalcoholic steatohepatitis, fatty liver degeneration or other diet-induced high fat or alcohol-induced tissue-degenerative conditions, cirrhosis, liver failure, or alcoholic hepatitis.

In one embodiment, the invention provides compound 121(*S*), for use in treating a diabetes-related disease or condition. This disease may be selected from insulin resistance, retinopathy, diabetic ulcers, radiation-associated necrosis, acute kidney failure or drug-induced nephrotoxicity.

In one embodiment, the invention provides compound 121(*S*), for use in treating a patient suffering from cystic fibrosis, including those patients suffering from chronic Pseudomonas bronchitis.

In one embodiment, the invention provides compound 121(*S*), for use in treating wounds. Examples of types of wounds that may be treated include venous ulcers, diabetic ulcers and pressure ulcers.

In one embodiment, the invention provides compound 121(*S*), for use in treating a disease or condition in a patient in need thereof selected from insulin dependent diabetes; non-insulin dependent diabetes; metabolic syndrome; obesity; insulin resistance; dyslipidemia; pathological glucose tolerance; hypertension; hyperlipidemia; hyperuricemia; gout; and hypercoagulability.

In one embodiment, the invention provides compound 121(*S*), for use in treating a disease or condition in a patient in need thereof wherein the disease or condition is selected from anemia, Graves disease, retinal vein occlusion, lupus nephritis, macular degeneration, myelodysplasia, pruritis of HIV origin, pulmonary hypertension, retinal artery occlusion, intestinal inflammation, ischemic optic neuropathy, acute pancreatitis, sickle cell anemia and beta thalassemia.

Medical uses delineated herein also include those wherein the patient is identified as in need of a particular stated treatment. Identifying a patient in need of such treatment can be in the judgment of a patient or a health care professional and can be subjective (e.g. opinion) or objective (e.g. measurable by a test or diagnostic method).

In another embodiment, any of the above medical uses comprises one or more second therapeutic agents. The choice of second therapeutic agent may be made from any second therapeutic agent known to be useful for co-administration with pentoxifylline. The choice of second therapeutic agent is also dependent upon the particular disease or condition to be treated. Examples of second therapeutic agents that may be employed in the methods of this invention are those set forth above for use in combination compositions comprising a compound of this invention and a second therapeutic agent.

In particular, the combination therapies of this invention include compound 121(*S*) and a second therapeutic agent for use in treatment of the following conditions (with the particular second therapeutic agent indicated in parentheses following the indication): late radiation induced injuries (α-tocopherol), radiation-induced fibrosis (α-tocopherol), radiation induced lymphedema (α-tocopherol), chronic breast pain in breast cancer patients (α-tocopherol), type 2 diabetic nephropathy (captopril), malnutrition-inflammation-cachexia syndrome (oral nutritional supplement, such as Nepro; and oral anti-inflammatory module, such as Oxepa); and brain and central nervous system tumors (radiation therapy and hydroxyurea).

The combination therapies of this invention also include compound 121(*S*) and a second therapeutic agent for use in treatment of insulin dependent diabetes; non-insulin dependent diabetes; metabolic syndrome; obesity; insulin resistance; dyslipidemia; pathological glucose tolerance; hypertension; hyperlipidemia; hyperuricemia; gout; and hypercoagulability.

The second therapeutic agent may be administered together with the compound of this invention as part of a single dosage form (such as a composition of this invention comprising a compound of the invention and an second therapeutic agent as described above) or as separate, multiple dosage forms. Alternatively, the additional agent may be administered prior to, consecutively with, or following the administration of a compound of this invention. In such combination therapy treatment, both the compounds of this invention and the second therapeutic agent(s) are administered by conventional methods. The administration of a composition of this invention, comprising both a compound of the invention and a second therapeutic agent, to a patient does not preclude the separate administration of that same therapeutic agent, any other second therapeutic agent or any compound of this invention to said patient at another time during a course of treatment.

Effective amounts of these second therapeutic agents are well known to those skilled in the art and guidance for dosing may be found in patents and published patent applications referenced herein, as well as in Wells et al., eds., Pharmacotherapy Handbook, 2nd Edition, Appleton and Lange, Stamford, Conn. (2000); PDR Pharmacopoeia, Tarascon Pocket Pharmacopoeia 2000, Deluxe Edition, Tarascon Publishing, Loma Linda, Calif. (2000), and other medical texts. However, it is well within the skilled artisan's purview to determine the second therapeutic agent's optimal effective-amount range.

In one embodiment of the invention, where a second therapeutic agent is administered to a subject, the effective amount of the compound of this invention is less than its effective amount would be where the second therapeutic agent is not administered. In another embodiment, the effective amount of the second therapeutic agent is less than its effective amount would be where the compound of this invention is not administered. In this way, undesired side effects associated with high doses of either agent may be minimized. Other potential advantages (including without limitation improved dosing regimens and/or reduced drug cost) will be apparent to those of skill in the art.

In yet another aspect, the invention provides the use of compound 121(*S*) alone or together with one or more of the above-described second therapeutic agents in the manufacture of a medicament, either as a single composition or as separate dosage forms, for treatment or prevention in a patient of a disease, disorder or symptom set forth above. Another aspect of the invention is compound 121(*S*) for use in the treatment or prevention in a patient of a disease, disorder or symptom thereof delineated herein.

### SYNTHETIC EXAMPLES

The synthetic examples below provide detailed procedures for making certain compounds of this invention. It will be apparent to one skilled in the art that further compounds of this invention may be prepared through the use of other reagents or intermediates by reference to these procedures and the schemes described above. The prepared compounds were analyzed by NMR, mass spectrometry, and/or elemental analysis as indicated. ¹HNMR were taken on a 300 MHz instrument, which was useful for determining deuterium incorporation. Unless otherwise stated, the absence of an NMR signal as noted in the examples below indicates a level of deuterium incorporation that is at least 90%.

### Example 1 (reference). Synthesis of 3-Methyl-7-(methyl-d₃)-1-(5-oxohexyl)-1H-purine-2,6(3H,7H)-dione (Compound 100).

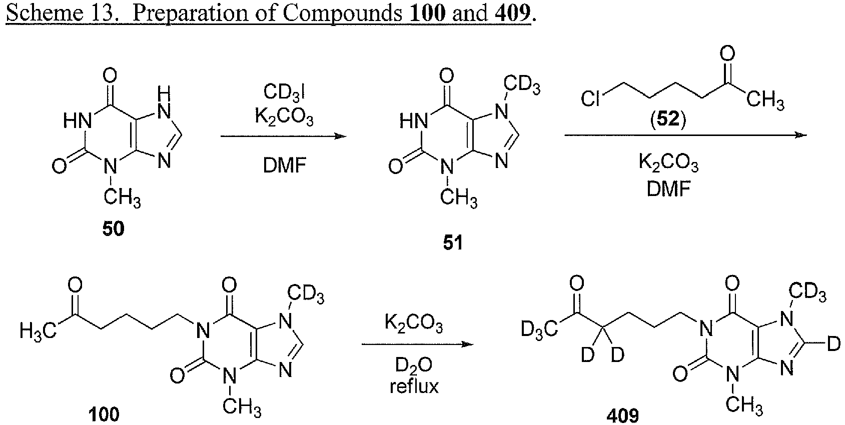

Step 1. 3-Methyl-7-(methyl-*d*₃)-1*H*-purine-2,6(3*H*,7*H*)-dione (51). A suspension of 3-methylxanthine **50** (5.0 g, 30.1 mmol, 1 equiv) and powdered K₂CO₃ (5.0 g, 36.0 mmol, 1.2 equiv) in DMF (95 mL) was heated to 60 °C and iodomethane-d₃ (Cambridge Isotopes, 99.5 atom% D, 2.2 mL, 36.0 mmol, 1.2 equiv) was added via syringe. The resulting mixture was heated at 80 °C for 5 hours (h). The reaction mixture was cooled to room temperature (rt) and the DMF was evaporated under reduced pressure. The crude residue was dissolved in 5% aqueous NaOH (50 mL), resulting in a dull yellow solution. The aqueous solution was washed with CH₂Cl₂three times (500 mL total). The aqueous layer was acidified to pH 5 with acetic acid (6 mL), resulting in formation of a tan precipitate. The mixture was cooled in an ice-water bath, and the solids were filtered and washed with cold water. The solid was dried in a vacuum oven to give 2.9 g of 51 as a tan solid. The filtrate was concentrated to approximately 25 mL and a second crop (0.70 g) of 51 was collected by filtration. The total yield of 51 was 3.6 g. The crude material was used without further purification.
Step 2. 3-Methyl-7-(methyl-*d₃*)-1-(5-oxohexyl-)1*H*-purine-2,6(3*H*,7*H*)-dione (Compound 100). Crude 51 (1.50 g, 8.2 mmol, 1 equiv) and powdered K₂CO₃ (2.28 g, 16.4 mmol, 2 equiv) were suspended in DMF (30 mL) and heated to 50 °C. To the resulting tan suspension was added 6-chloro-2-hexanone (52, 1.2 mL, 9.0 mmol, 1.1 equiv) and the reaction temperature was raised to 130 °C. Heating was continued at 130 °C for 2 h, during which time the suspension became finer and darker in color. The reaction mixture was cooled to rt and DMF was evaporated under reduced pressure. The residual tan paste was suspended in EtOAc (250 mL) and filtered to remove insoluble material. The filtrate was concentrated under reduced pressure resulting in a yellow oil. The crude product was purified using an Analogix chromatography system eluting with 100% EtOAc (10 minutes) followed by a gradient of 0 to 25% MeOH/EtOAc over 50 minutes (min). Product fractions were concentrated under reduced pressure to give a slightly yellow oil that solidified after standing for several minutes. The solid was triturated with heptanes (100 mL) and filtered to give 2.00 g of **100** as an off-white solid, mp 101.8-103.0 °C. ¹H-NMR (300 MHz, CDCl₃): δ 1.64-1.68 (m, 4H), 2.15 (s, 3H), 2.51 (t, J = 7.0, 2H), 3.57 (s, 3H), 4.01 (t, J = 7.0, 2H), 7.52 (s, 1H). ¹³C-NMR (75 MHz, CDCl₃): δ 20.95, 27.41, 29.69, 29.98, 40.80, 43.18, 107.63, 141.41, 148.75, 151.45, 155.26, 208.80. HPLC (method: 20 mm C18-RP column - gradient method 2 to 95% ACN + 0.1% formic acid in 3.3 min with 1.7 min hold at 95% ACN; Wavelength: 254 nm): retention time: 2.54 min; 98.5% purity. MS (M+H): 282.0. Elemental Analysis (C₁₃H₁₅D₃N₄O₃): Calculated: C=55.50, H=6.45, N=19.92. Found: C=55.58, H=6.48, N=19.76.

Due to the presence of a triplet at 4.01 ppm in the above ¹H-NMR spectrum, determination of the presence or absence of a singlet peak at around 3.99 ppm corresponding to the presence or absence of hydrogens on the N-methyl group at the 7 position (R¹) of the purine ring was not possible.

### Example 2 (reference). Synthesis of 8-d₁-3-methyl-7-(methyl-d₃)-1-(6-d₃-4-d₂-5-oxohexyl)-1H-purine-2,6(3H,7H)-dione (Compound 409).

8-*d₁*-3-methyl-7-(methyl-*d₃*)-1-(6-*d₃*-4-*d₂*-5-oxohexyl)-1*H*-purine-2,6(3*H*,7*H*)-dione (Compound **409).** A suspension of **100** (1.80 g, 6.4 mmol, 1 equiv) and powdered K₂CO₃ (0.23 g, 1.7 mmol, 0.25 equiv) in D₂O (Cambridge Isotope Labs, 99 atom% D) (45 mL) was stirred under reflux conditions for 24 h during which time the suspension became a slightly yellow solution. The reaction mixture was cooled to rt, saturated with sodium chloride, and extracted four times with dichloromethane (400 mL total). The combined organic solution was dried over Na₂SO₄, filtered, and evaporated under reduced pressure to provide 1.7 g of a slightly yellow oil that solidified upon standing. The crude material was re-subjected to the hydrogen/deuterium exchange conditions described above with fresh K₂CO₃ and D₂O. After an identical workup, the off-white solid was triturated with hexanes (100 mL) and filtered to give 1.61 g of **409** as an off white solid, mp 99.6-99.8 °C. ¹H-NMR (300 MHz, CDCl₃): δ 1.64-1.69 (m, 4H), 3.57 (s, 3H), 4.01 (t, J = 7.0, 2H). ¹³C-NMR (75 MHz, CDCl₃): δ 21.05, 27.61, 29.90, 41.02, 107.83, 148.99, 151.69, 155.50, 209.28. HPLC (method: Waters Atlantis T3 2.1 x 50 mm 3 µm C18-RP column - gradient method 5-95% ACN + 0.1% formic acid in 14 min (1.0 mL/min) with 4 min hold at 95% ACN; Wavelength: 254 nm): retention time: 3.26 min; 98% purity. MS (M+H): 288.3. Elemental Analysis (C₁₃H₉D₉N₄O₃): Calculated: C=54.35, H=6.31, N=19.50. Found: C=54.36, H=6.32, N=19.10.

Notable in the ¹H-NMR spectrum above was the absence of the following peaks: a singlet at around 2.15 ppm indicating an absence of methyl ketone hydrogens; a triplet at around 2.51 ppm indicating an absence of methylene ketone hydrogens; and a singlet at around 7.52 ppm indicating an absence of hydrogen at the number 8 position on the purine ring. Due to the presence of a triplet at 4.01 ppm in the above ¹H-NMR spectrum, determination of the presence or absence of a singlet peak at around 3.99 ppm corresponding to the presence or absence of hydrogens on the N-methyl group at the 7 position (R¹) of the purine ring was not possible.

The H/D exchange reaction to convert the CH₃C(O)CH₂ functional group in **100** to the CD₃C(O)CD₂ functional group in **409** may be generally applied under analogous conditions to convert compounds having one or more hydrogens *alpha* to the carbonyl group to compounds having one or more deuteriums in place of the corresponding one or more hydrogens. In one embodiment, successive H/D exchange reactions may be performed as needed to further increase the amount of deuterium incorporation. In one aspect of this embodiment, any excess D₂O at the end of a second H/D exchange reaction in a given batch run may be used in a first H/D exchange reaction in a subsequent batch run; and any excess D₂O at the end of a third H/D exchange reaction in a given batch run may be used in a second H/D exchange reaction in a subsequent batch run.

The Table below shows an exemplary deuteration on three separate batches of a compound having a CH₃C(O)CH₂ functional group and indicates the deuteration agent (either ≥99% D₂O or an aqueous phase obtained from a later deuteration cycle of another batch) that may be used according to this invention.

| | Deuteration Cycle 1 | Deuteration Cycle 2 | Deuteration Cycle 3 |
|---|---|---|---|
| Batch 1 | ≥99% D₂O | ≥99% D₂O | ≥99% D₂O |
| Batch 2 | (a) Aqueous phase from Batch 1, Deuteration Cycle 2 or | Aqueous phase from Batch 1, Deuteration Cycle 3 | ≥99% D₂O |
| | (b) Aqueous phase from Batch 1, Deuteration Cycle 3 | | |
| Batch 3 | (a) Aqueous phase from Batch 2, Deuteration Cycle 2 or | (a) Aqueous phase from Batch 1, Deuteration Cycle 3 or | ≥99% D₂O |
| | (b) Aqueous phase from Batch 2, Deuteration Cycle 3 or | (b) Aqueous phase from Batch 2, Deuteration Cycle 3 | |
| | (c) Aqueous phase from Batch 1, Deuteration Cycle 2 or | | |
| | (d) Aqueous phase from Batch 1, Deuteration Cycle 3 | | |

By way of example, the conversion of pentoxifylline 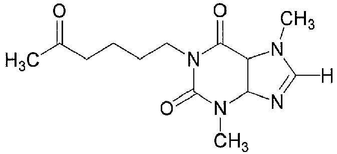 to the structure below 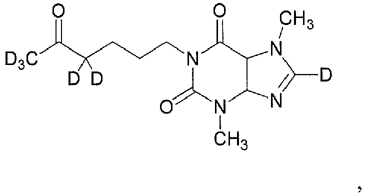 may be effected in successive batches. The following table shows the percentage of deuterium incorporation at each of the methyl(CO), (CO)methylene, and the imidazole ring methine carbon for successive batch runs of 50 kg of pentoxifylline in each batch:

| Batch 1 | (CO)methylene | methyl(CO) | methine carbon |
|---|---|---|---|
| Exchange 1 | 88.6 | 88.8 | 13.8 |
| Exchange 2 | 97.8 | 98.0 | 26.3 |
| Exchange 3 | 99.2 | 99.2 | 37.4 |

| Batch 2 | | | |
|---|---|---|---|
| Exchange 1 (R1-2) | 82.3 | 83.8 | 6.4 |
| Exchange 2 (R1-3) | 96.7 | 96.8 | 12.4 |
| Exchange 3 | 98.9 | 98.9 | 26.4 |

| Batch 3 | | | |
|---|---|---|---|
| Exchange 1 (R2-2) | 78.0 | 80.8 | 5.8 |
| Exchange 2 (R2-2) | 95.1 | 95.6 | 11.6 |
| Exchange 3 | 98.9 | 99.0 | 27.1 |
| Exchange 4* | 99.3 | 99.4 | 32.6 |

| | | | |
|---|---|---|---|
| R1-2: recycled from Batch 1, Exchange 2 R1-3: recycled from Batch 1, Exchange 3 R2-2: recycled from Batch 2, Exchange 2 R2-3: recycled from Batch 2, Exchange 3 | | | |

In one embodiment, Exchange 4 is optional. In the Batch 3 run shown in the table, Exchange 4 was performed at half-volume to ensure high deuterium incorporation in Batch 3.

### Example 8 (reference). Synthesis of (±)8-d₁-1-(4,4,5,6,6,6-d₆-5-Hydroxyhexyl)-3-methyl-7-(methyl-d₃)-1H-purine-2,6(3H,7H)-dione (Compound 435).

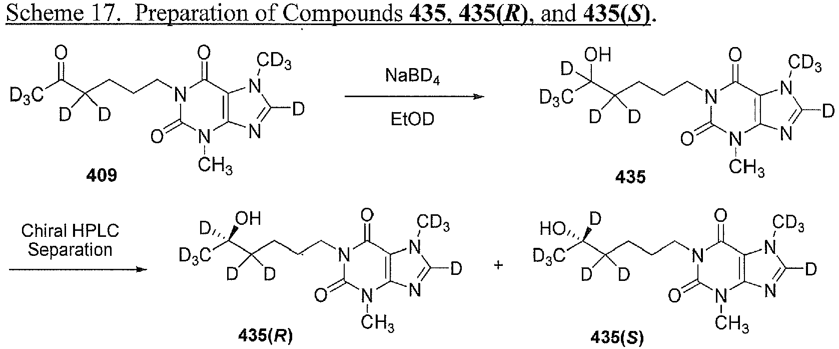

(±)8-*d₁*-1-(4,4,5,6,6,6-*d₆*-5-Hydroxyhexyl)-3-methyl-7-(methyl-*d₃*)-1*H-*purine-2,6(3*H*,7*H*)-dione (Compound **435**). To a solution of Compound 409 (0.50 g, 1.7 mmol, 1.0 equiv) in EtOD (13 mL, Aldrich 99.5 atom% D) was added NaBD₄ (0.08 g, 1.9 mmol, 1.1 equiv, Cambridge Isotope Labs, 99 atom% D). An increase in temperature from 24 to 27 °C was observed. The reaction was stirred 2 hours at room temperature then was quenched by the addition of of D₂O (30 mL) (Cambridge Isotope, 99 atom% D). A white suspension formed that was extracted with MTBE (4X, 200 mL total). The combined organic extracts were dried over Na₂SO₄, filtered, and concentrated under reduced pressure to a clear, colorless oil (0.45 g). The crude product was purified by silica gel chromatography eluting first with 1% MeOH/ CH₂Cl₂ followed by a gradient of 1-5% MeOH/ CH₂Cl₂. Fractions containing product were concentrated under reduced pressure to give 0.40 g (81 %) of Compound **435** as a clear colorless oil that solidified on standing.

### Example 9 (reference). Chiral Separation of (R)-8-d₁-1-(4,4,5,6,6,6-d₆-5-Hydroxyhexyl)-3-methyl-7-(methyl-d₃)-1H-purine-2,6(3H,7H)-dione (Compound 435(R)) and (S)-8-d₁-1-(4,4,5,6,6,6-d₆-5-Hydroxyhexyl)-3-methyl-7-(methyl-d₃)-1H-purine-2,6(3H,7H)-dione (Compound 435(S)).

Separation of Enantiomers of Compound **435.** Compound **435** obtained from Example 8 above (0.32 g) was dissolved in a minimal amount of iPrOH (5 mL, HPLC grade, heating was required) and diluted with hexane (4 mL, HPLC grade). Enantiomer separation was achieved using a Waters HPLC system equipped with a preparative Daicel Chiralpak AD column (20 X 250 mm). For the first minute of the run, the mobile phase was 80% hexane and 20% iPrOH along with 0.1% diethylamine. After the first minute a gradient to 75% hexane and 25% iPrOH along with 0.1% diethylamine over 15 minutes was used, followed by holding at this solvent ratio for 17 minutes at a flow rate of 18 mL/min. This method resulted in baseline separation with Compound **435(*R*)** eluting first (21.9 min), followed by Compound **435(*S*)** (25.2 min). Fractions containing each enantiomer were concentrated under reduced pressure to give 0.12 g each of **435(R)** (mp 108.0-108.1 °C) and **435(*S*)** (mp107.6-107.7 °C) as off-white solids.

A). (*R*)-8-*d₁*-1-(4,4,5,6,6,6-*d₆*-5-Hydroxyhexyl)-3-methyl-7-(methyl-*d₃*)-1*H-*purine-2,6(3*H*,7*H*)-dione (Compound **435(*R*)**).¹H-NMR (300 MHz, CDCl₃): δ 1.40-1.48 (m, 3H), 1.66-1.70 (m, 2H), 3.58 (s, 3H), 4.02 (t, J = 7.5, 2H). ¹³C-NMR (75 MHz, CDCl₃): δ 22.66, 27.86, 29.71, 41.15, 107.67, 148.80, 151.54, 155.41. HPLC (method: Waters Atlantis T3 2.1 x 50 mm 3 µm C18-RP column - gradient method 5-95% ACN + 0.1% formic acid in 14 min (1.0 mL/min) with 4 min hold at 95% ACN + 0.1% formic acid; Wavelength: 254 nm): retention time: 3.25 min; 99.8% purity. Chiral HPLC (method: Chiralpak AD 25 cm column - isocratic method 78% hexane/ 22% isopropanol/0.01% diethylamine for 40 min at 1.00 mL/min; Wavelength: 254 nm): retention time: 27.24 min (major enantiomer); 31.11 min (expected for minor enantiomer): >99.9% *ee* purity. MS (M+H): 291.3, (M+Na): 313.2. Elemental Analysis (C₁₃H₁₀D₁₀N₄O₃): Calculated: C=53.78, H=6.94, N=19.30. Found: C=54.01, H=7.07, N=18.90.

Notable in the ¹H-NMR spectrum above was the absence of the following peaks: a peak at around 1.19 ppm indicating an absence of methyl hydrogens alpha to the hydroxyl group; a peak at around 3.80 ppm indicating an absence of hydrogen at the methinyl hydroxyl position; and a singlet at around 7.51 ppm indicating an absence of hydrogen at the number 8 position on the purine ring. Due to the presence of a multiplet at 1.36-1.50 ppm and a triplet at 4.01 ppm in the above ¹H-NMR spectrum, determination of the presence or absence a peak at 1.51 ppm corresponding to the presence or absence of methylene hydrogens alpha to the hydroxyl group and of a singlet peak at around 3.99 ppm corresponding to the presence or absence of hydrogens on the N-methyl group at the 7 position (R¹) of the purine ring was not possible.

B). (*S*)-8-*d₁*-1-(4,4,5,6,6,6-*d₆*-5-Hydroxyhexyl)-3-methyl-7-(methyl-*d₃*)-1*H-*purine-2,6(3*H*,7*H*)-dione (Compound **435(*S*)**). ¹H-NMR (300 MHz, CDCl₃): δ 1.41-1.48 (m, 3H), 1.62-1.72 (m, 2H), 3.58 (s, 3H), 4.03 (t, J = 7.4, 2H). ¹³C-NMR (75 MHz, CDCl₃): δ 22.69, 27.90, 29.70, 41.17, 107.69, 148.82, 151.58, 155.43. HPLC (method: Waters Atlantis T3 2.1 x 50 mm 3 µm C18-RP column - gradient method 5-95% ACN + 0.1% formic acid in 14 min (1.0 mL/min) with 4 min hold at 95% ACN + 0.1% formic acid; Wavelength: 254 nm): retention time: 3.25 min; 99.5% purity. Chiral HPLC (method: Chiralpak AD 25 cm column - isocratic method 78% hexane/ 22% isopropanol/0.01 % diethylamine for 40 min at 1.00 mL/min; Wavelength: 254 nm): retention time: 31.11 min (major enantiomer); 27.24 min (expected for minor enantiomer): >99.9% *ee* purity. MS (M+H): 291.3, (M+Na): 313.2. Elemental Analysis (C₁₃H₁₀D₁₀N₄O₃): Calculated: C=53.78, H=6.94, N=19.30. Found: C=54.01, H=7.11, N=18.78.

Notable in the ¹H-NMR spectrum above was the absence of the following peaks: a peak at around 1.19 ppm indicating an absence of methyl hydrogens alpha to the hydroxyl group; a peak at around 3.80 ppm indicating an absence of hydrogen at the methinyl hydroxyl position; and a singlet at around 7.51 ppm indicating an absence of hydrogen at the number 8 position on the purine ring. Due to the presence of a multiplet at 1.36-1.50 ppm and a triplet at 4.01 ppm in the above ¹H-NMR spectrum, determination of the presence or absence a peak at 1.51 ppm corresponding to the presence or absence of methylene hydrogens alpha to the hydroxyl group and of a singlet peak at around 3.99 ppm corresponding to the presence or absence of hydrogens on the N-methyl group at the 7 position (R¹) of the purine ring was not possible.

### Example 10 (reference). Synthesis of 8-d₁-3,7-Dimethyl-1-(4,4,6,6,6-d₆-5-oxohexyl)-1H-purine-2,6(3H,7H)-dione Compound 407).

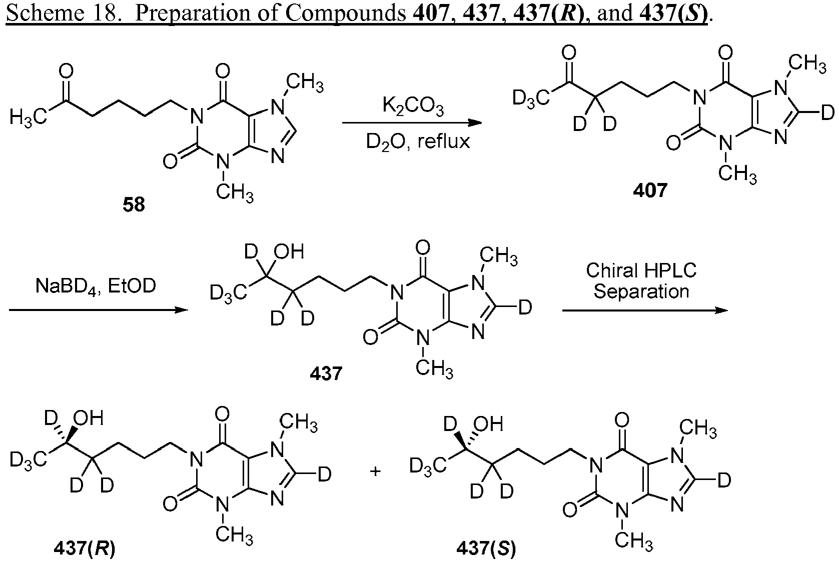

8-*d₁*-3,7-Dimethyl-1-(4,4,6,6,6-*d₅*-5-oxohexyl)-1*H*-purine-2,6(3*H*,7*H*)-dione (Compound **407).** A mixture of commercially-available **58** (7.95 g, 28.6 mmol) and potassium carbonate (990 mg, 7.2 mmol) in D₂O (195 mL, Cambridge Isotopes, 99.9 atom% D) was heated to reflux for 24 hours. The suspended solid dissolved gradually giving a yellow solution. The solution was cooled to approximately 40 °C and was concentrated under reduced pressure to a tan solid. The solid was dissolved in D₂O (195 mL) and the solution was heated to reflux for another 24 hours. The solution was cooled to room temperature and concentrated under reduced pressure to a tan solid. Ethyl acetate (200 mL) was added and the mixture was stirred 0.5 hours at approximately 40 °C. The insoluble materials were filtered off and the filtrate was concentrated under reduced pressure to a pale yellow solid, which was triturated with MTBE (40 mL) to give 7.5 g (93%) of Compound **407** as an off-white solid. ¹H-NMR (300 MHz, CDCl₃): δ 1.64-1.68 (m, 4H), 3.57 (s, 3H), 3.99 (s, 3H), 3.99-4.04 (m, 2H). ¹³C-NMR (75 MHz, CDCl₃): δ 20.84, 27.40, 29.69, 33.57, 40.81, 107.62, 148.77, 151.48, 155.28, 209.07. HPLC (method: Waters Atlantis T3 2.1 x 50 mm 3 µm C18-RP column - gradient method 5-95% ACN + 0.1% formic acid in 14 min (1.0 mL/min) with 4 min hold at 95% ACN + 0.1% formic acid; Wavelength: 305 nm): retention time: 3.24 min; 99.9% purity. MS (M+H): 285.3, (M+Na): 307.2. Elemental Analysis (C₁₃H₁₂D₆N₄O₃): Calculated: C=54.92, H=6.38, N=19.71. Found: C=54.89, H=6.38, N=19.70.

Notable in the ¹H-NMR spectrum above was the absence of the following peaks: a singlet at around 2.15 ppm indicating an absence of methyl ketone hydrogens; a triplet at around 2.51 ppm indicating an absence of methylene ketone hydrogens; and a singlet at around 7.52 ppm indicating an absence of hydrogen at the number 8 position on the purine ring.

### Example 11 (reference). Synthesis of (±)8-d₁-1-(4,4,5,6,6,6-d₆-5-Hydroxyhexyl)-3,7-dimethyl-1H-purine-2,6(3H,7H)-dione (Compound 437).

(±)8-*d₁*-1-(4,4,5,6,6,6-*d₆*-5-Hydroxyhexyl)-3,7-dimethyl-1H-purine-2,6(3H,7H)-dione (Compound **437).** Sodium borodeuteride (1.06 g, 25.3 mmol, Cambridge Isotopes, 99 atom% D) was added to a suspension of **407** (6.5 g, 22.9 mmol) in ethanol-d₁ (65 mL, Aldrich, 99.5 atom% D) at 0 °C. The mixture was warmed to room temperature and stirred until a clear solution had developed (approximately 1 hour). The reaction was quenched with a saturated solution of ammonium chloride-d₄ (Cambridge Isotopes, 98 atom% D) in D₂O (8 mL, Cambridge Isotope, 99.9 atom% D), ethanol-d₁ was evaporated under reduced pressure and the residue was extracted with EtOAc (160 mL). The organic phase was washed with D₂O (20 mL), dried over sodium sulfate, filtered and concentrated under reduced pressure to give 4.8 g (73%) of Compound **437** as a pale yellow solid.

### Example 12 (reference). Chiral Separation of (R)-8-d₁-1-(4,4,5,6,6,6-d₆-5-Hydroxyhexyl)-3,7-dimethyl-1H-purine-2,6(3H,7H)-dione Compound 437(R)) and (S)-8-d₁-1-(4,4,5,6,6,6-d₆-5-Hydroxyhexyl)-3,7-dimethyl-1H-purine-2,6(3H,7H)-dione (Compound 437(S)).

Separation of Enantiomers of Compound **437.** Compound **437** obtained from Example 11 above (1.60 g) was dissolved in iPrOH (20 mL, HPLC grade, heating required). Enantiomeric separation was achieved using a Waters HPLC system equipped with a preparative Chiralpak AD column (20 x 250 mm Daicel, 10 µM) with a preparative Chiralpak AD guard column (20 x 50 mm Daicel, 10 µM) preceding it. For the first minute of the run, the sample was eluted with 20% iPrOH/hexanes (henceforth, with 0.1% diethylamine as co-eluent) while ramping up from a flow rate of 15 mL/min to 18 mL/min. Over the next 15 minutes, the sample was eluted at a flow rate of 18 mL/min with a gradient of 20% to 25% iPrOH/hexanes. For the next 19 minutes the sample was eluted at a flow rate of 18 mL/min with 25% iPrOH/hexanes. Over the next 0.5 minutes, the sample was eluted at a flow rate of 18 mL/min with a gradient of 25% to 20% iPrOH/hexanes. For the next 4.5 minutes, the sample was eluted at a flow rate of 18 mL/min with 20% iPrOH/hexanes. This elution method resulted in baseline separation of Compound **437(*R*)** eluting first (retention time approximately 29 min) and Compound **437(*S*)** eluting second (retention time approximately 33 min). Fractions containing each enantiomer were collected and concentrated under reduced pressure to give 340 mg of **437(*R*)** (mp 112.0-114.5 °C) and 375 mg of **437(*S*)** (mp 111.9-112.3 °C) as off-white solids. [Note: only 1.0 g of **437** was injected from the solution prepared above.]

A. (*R*)-8-*d₁*-1-(4,4,5,6,6,6-*d₆*-5-Hydroxyhexyl)-3,7-dimethyl-1H-purine-2,6(3H,7H)-dione (Compound **437(*R*)**). ¹H-NMR (300 MHz, CDCl₃): δ 1.36-1.50 (m, 2H), 1.54 (s, 1H), 1.64-1.74 (m, 2H), 3.58 (s, 3H), 3.99 (s, 3H), 4.00-4.05 (m, 2H). ¹³C-NMR (75 MHz, CDCl₃): δ 22.66, 27.86, 29.70, 33.59, 41.14, 107.65, 148.76, 151.52, 155.40. HPLC (method: Waters Atlantis T3 2.1 x 50 mm 3 µm C18-RP column - gradient method 5-95% ACN + 0.1% formic acid in 14 min (1.0 mL/min) with 4 min hold at 95% ACN + 0.1% formic acid; Wavelength: 305 nm): retention time: 3.28 min; 99.9% purity. Chiral HPLC (method: Chiralpak AD 25 cm column - isocratic method 78% hexane/ 22% isopropanol/0.01% diethylamine for 40 min at 1.00 mL/min; Wavelength: 254 nm): retention time: 25.20 min (major enantiomer); 28.39 min (expected for minor enantiomer): >99.9% *ee* purity. MS (M+H): 288.3, (M+Na): 310.2. Elemental Analysis (C₁₃H₁₃D₇N₄O₃): Calculated: C=54.34, H=7.02, N=19.50. Found: C=54.32, H=7.23, N=19.35.

Notable in the ¹H-NMR spectrum above was the absence of the following peaks: a peak at around 1.19 ppm indicating an absence of methyl hydrogens alpha to the hydroxyl group; a peak at around 3.80 ppm indicating an absence of hydrogen at the methinyl hydroxyl position; and a singlet peak at around 7.51 ppm indicating an absence of hydrogen at the number 8 position on the purine ring. Due to the presence of a multiplet at 1.36-1.50 ppm in the above ¹H-NMR spectrum, determination of the presence or absence a peak at 1.51 ppm corresponding to the presence or absence of methylene hydrogens alpha to the hydroxyl group was not possible.

B. (*S*)-8-*d₁*-1-(4,4,5,6,6,6-*d₆*-5-Hydroxyhexyl)-3,7-dimethyl-1H-purine-2,6(3H,7H)-dione (Compound **437(*S*)**). ¹H-NMR (300 MHz, CDCl₃): δ 1.38-1.48 (m, 2H), 1.55 (s, 1H), 1.64-1.72 (m, 2H), 3.58 (s, 3H), 3.99 (s, 3H), 4.00-4.05 (m, 2H). ¹³C-NMR (75 MHz, CDCl₃): δ 22.65, 27.84, 29.71, 33.59, 41.13, 107.64, 148.75,151.52,155.39. HPLC (method: Waters Atlantis T3 2.1 x 50 mm 3 µm C18-RP column - gradient method 5-95% ACN + 0.1% formic acid in 14 min (1.0 mL/min) with 4 min hold at 95% ACN + 0.1% formic acid; Wavelength: 305 nm): retention time: 3.27 min; 99.9% purity. Chiral HPLC (method: Chiralpak AD 25 cm column - isocratic method 78% hexane/ 22% isopropanol/0.01% diethylamine for 40 min at 1.00 mL/min; Wavelength: 254 nm): retention time: 28.39 min (major enantiomer); 25.20 min (expected for minor enantiomer): >99.9% *ee* purity. MS (M+H): 288.3, (M+Na): 310.2. Elemental Analysis (C₁₃H₁₃D₇N₄O₃): Calculated: C=54.34, H=7.02, N=19.50. Found: C=54.33, H=7.30, N=19.36.

Notable in the ¹H-NMR spectrum above was the absence of the following peaks: a peak at around 1.19 ppm indicating an absence of methyl hydrogens alpha to the hydroxyl group; a peak at around 3.80 ppm indicating an absence of hydrogen at the methinyl hydroxyl position; and a singlet peak at around 7.51 ppm indicating an absence of hydrogen at the number 8 position on the purine ring. Due to the presence of a multiplet at 1.36-1.50 ppm in the above ¹H-NMR spectrum, determination of the presence or absence a peak at 1.51 ppm corresponding to the presence or absence of methylene hydrogens alpha to the hydroxyl group was not possible.

### Example 13 (reference): Alternative synthesis of (S)-8-d₁-1-(4,4,5,6,6,6-d₆-5-Hydroxyhexyl)-3,7-dimethyl-1H-purine-2,6(3H,7H)-dione (Compound 437(S)).

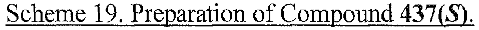

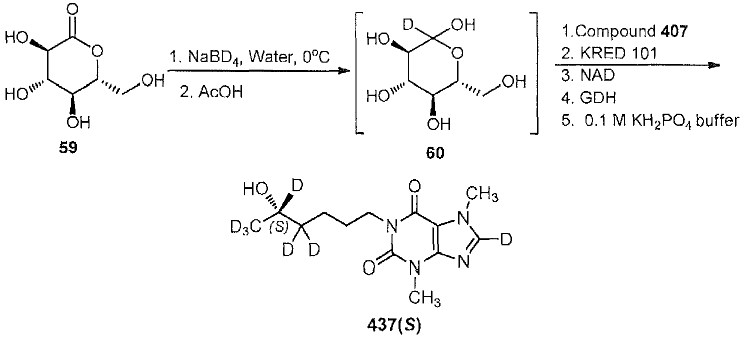

*(S)-*8*-d₁**-*1*-*(4,4,5,6,6,6-*d₆*-5-Hydroxyhexyl)-3,7-dimethyl-1H-purine-2,6(3H,7H)-dione (Compound **437(*S*)),** D-Glucono-δ-lactone **59** (5 g, 28.09 mmoles) was added in one portion to ice-cold water (35 mL, 0-3°C) and stirred for 10 min. A freshly prepared, ice-cold solution of NaBD₄ (0.294 g, 7.02 mmoles, 99%D) in 10 mL of water was added slowly over 10 min. The reaction is slightly exothermic (2° to 10°C) and the pH of the reaction is 7.42. Stirring was continued for 30 min, with the temperature maintained at 0-3°C by cooling. Acetic acid (0.32 mL, 5.61 mmoles) was added and stirring was continued for another 30 min.

The reaction mixture was diluted with 18 mL of water and the solution was heated to 25-30°C. KH₂PO₄ (0.85 g) was added to the mixture and the pH was adjusted to 7 with 4M KOH solution. To the resulting mixture was added (2.5 g, 8.8 mmoles) of Compound **407.** A solution of NAD (15 mg), GDH (2.5 mg), KRED 101 (25 mg) in 12.5 mL of 0.1 KH₂PO₄ buffer was added. The resulting solution was stirred at 25-30°C. The pH of the reaction mixture was maintained between 6 and 7 by adding 4M KOH solution drop-wise as needed. HPLC monitoring of the reaction indicated that the reaction was complete after 12 hours with 99.97A% conversion by HPLC analysis. Sodium chloride (12.5 g) was added and stirred for 30 min. The mixture was extracted with ethyl acetate (3 x 25 mL). The organic layer was separated, filtered through a celite pad and concentrated to a small volume (∼ 5 vol). Product solids precipitated during the concentration. The slurry was heated at 40-60 °C and heptanes (20 mL) was added over 10 minutes. The slurry was stirred overnight at 20-25 °C and filtered. The wet cake dried at 50 °C for 12 hours to afford Compound **437(*S*)** as a white solid. (2.12 g, 85% yield). The product purity was determined to be >99.5A% by HPLC analysis. A single enantiomer was observed by chiral HPLC analysis. The deuterium incorporation at the methine 5 position was ∼95% D. HPLC (method: Waters Symmetry 4.6 x 50 mm 3.5 µm C18 column - gradient method: 15% MeOH + 85% 0.1% formic acid in water for 5 min (1.25 mL/min), ramp to 80% MeOH + 20% 0.1% formic acid in water over 5 min, ramp to 15% MeOH + 85% 0.1% formic acid in water over 6 s followed by a 3.9 min hold at 15% MeOH + 85% 0.1% formic acid in water; wavelength: 274 nm): >99.5% purity. Chiral HPLC analysis (method: Chiralpak AD-H 25 cm column - isocratic method 75% *n*-heptane/ 25% isopropanol for 25 min at 1.25 mL/min; wavelength: 274 nm): retention time: 17.56 min (major enantiomer); 15.5 min (expected for minor enantiomer): >99.95% *ee* purity.

### Example 17 (reference). Synthesis of (±) 1-(4,4,6,6,6-d₅-5-hydroxyhexyl)-3,7-dimethyl-8-d-1H-purine-2,6(3H,7H)-dione (Compound 421).

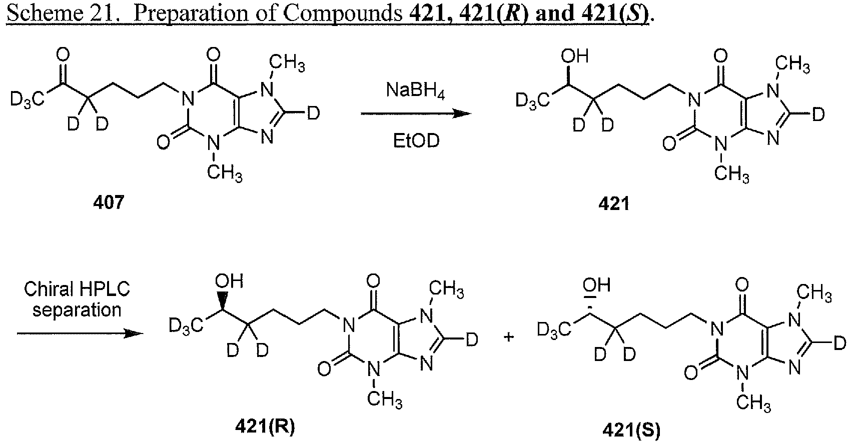

Synthesis of (±) 1-(4,4,6,6,6-d₅-5-hydroxyhexyl)-3,7-dimethyl-8-d-1*H-*purine-2,6(3*H*,7*H*)-dione (Compound **421).** Following the same general method as for the synthesis of Compound **437** in Example 11 above, Compound **407** (see Example 10) was treated with NaBH₄ in EtOD and extracted with CH₂Cl₂ to afford Compound **421.**

### Example 18 (reference). Chiral Separation of (R)-1-(4,4,6,6,6-d₅-5-hydroxyhexyl)-3,7-dimethyl-8-d-1H-purine-2,6(3H,7H)-dione (Compound 421(R)) and (S)-1-(4,4,6,6,6-d₅-5-hydroxyhexyl)-3,7-dimethyl-8-d-1H-purine-2,6(3H,7H)-dione (Compound 421(S)).

Separation of Enantiomers **421(*R*)** and **421(*S*)** from (±)Compound **421.** A portion of racemic Compound **421** obtained as described above was separated in the same manner as racemic Compound **437** (see Example 12) to afford separated enantiomers Compound **421(*R*)** (560 mg) and Compound **421(*S*)** (520 mg).

A. (*R*)-1-(4,4,6,6,6-d₅-5-hydroxyhexyl)-3,7-dimethyl-8-d-1*H-*purine-2,6(3*H*,7*H*)-dione (Compound **421(*R*))**. ¹H-NMR (300 MHz, CDCl₃): δ 1.41-1.48 (m, 2H), 1.64-1.72 (m, 3H), 3.58 (s, 3H), 3.79 (s, 1H), 3.99 (s, 3H), 4.03 (t, *J*=7.3, 2H). ¹³C-NMR (75 MHz, CDCl₃): δ 22.69, 27.84, 29.72, 33.60, 41.14, 67.62, 107.64, 148.74, 151.51, 155.38. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 254 nm): retention time: 3.33 min; >99.9% purity. Chiral HPLC (method: Chiralpak AD 25 cm column - isocratic method 78% hexane/ 22% isopropanol/0.1% diethylamine for 40 minutes at 1.00 mL/min; Wavelength: 254 nm): retention time: 24.77 min (R enantiomer); 28.16 min (expected for S enantiomer); >99.9% *ee* purity. MS (M+H-H₂O): 269.1; (M+H): 287.1; (M+Na): 309.3. Elemental Analysis (C₁₃H₁₄D₆N₄O₃): Calculated: C=54.53, H=7.04, N=19.57. Found: C=54.44, H=7.18, N=19.32.

B. (*S*)-1-(4,4,6,6,6-d₅-5-hydroxyhexyl)-3,7-dimethyl-8-d-1*H*-purine-2-6(3*H*,7*H*)-dione (Compound **421(*S*))**. ¹H-NMR (300 MHz, CDCl₃): δ 1.37-1.48 (m, 2H), 1.64-1.74 (m, 3H), 3.58 (s, 3H), 3.79 (s, 1H), 3.99 (s, 3H), 4.03 (t, *J*=7.4, 2H). ¹³C-NMR (75 MHz, CDCl₃): δ 22.70, 27.84, 29.71, 33.60, 41.14, 67.61, 107.64, 148.74, 151.51, 155.38. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 254 nm): retention time: 3.34 min; >99.9% purity. Chiral HPLC (method: Chiralpak AD 25 cm column - isocratic method 78% hexane/ 22% isopropanol/0.1% diethylamine for 40 minutes at 1.00 mL/min; Wavelength: 254 nm): retention time: 28.16 min (S enantiomer); 24.77 min (expected for R enantiomer); >99.9% *ee* purity. MS (M+H-H₂O): 269.1; (M+H): 287.1; (M+Na): 309.3. Elemental Analysis (C₁₃H₁₄D₆N₄O₃): Calculated: C=54.53, H=7.04, N=19.57. Found: C=54.54, H=7.18, N=19.31.

Notable in the ¹H-NMR spectrum of both **421(*R*)** and **421(*S*)** was the absence of a peak at around 7.51 ppm, indicating an absence of hydrogen at the 2-position on the imidazole ring system.

### Alternative preparation of 421(R):

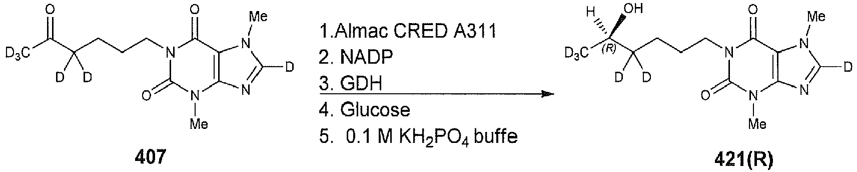

### Preparation of Compound 421(R) from Compound 407 Using CRED A131

A 100mL 3-necked RB flask equipped with a heating mantle, a J-Kem thermocouple, magnetic stir bar, a reflux condenser, and a pH probe was charged with 407 (500 mg, 1.75 mmol), D(+) Glucose (750 mg, 1.5 wt) in 10 mL buffer (0.1M KH₂PO₄, pH = 7.0) and heated to 25-30°C. A solution ofNADP (15 mg, 3 wt%), GDH (3 mg, 0.6 wt%), ALMAC CRED A311-NADP (30 mg, 6 wt%) in 0.1 M KH₂PO₄ buffer was added and maintained reaction temperature 25-30°C. To this added 1mL of methyl-*t*-butyl ether (MTBE). The pH of the reaction mixture was maintained between 6 and 7 adding 4M KOH solution drop-wise. The reaction was monitored by HPLC and was complete after 29 hours with 99.87A% conversion by HPLC. Sodium chloride (2.5 g, 5 wt) was added and stirred for 20 min. The reaction mixture was extracted with ethyl acetate (3 x 15 mL). The organic layer was separated, filtered through celite pad and concentrated to a small volume (∼ 5 vol) and product solids were precipitated. Heptanes (5 mL) was added to the slurry (at 40-60°C) over 5 minutes. The slurry was stirred at 20-25°C and filtered. The wet cake was dried at 50°C for 12 hours to afford **421(*R*)** as a white solid. (0.422 g, 84% yield). The isolated product purity was >99.5% by HPLC and single enantiomer by chiral HPLC.

### Example 19 (reference). Synthesis of (±)-1-(4,4,5,6,6,6-d₆-5-Hydroxyhexyl)-3,7-dimethyl-1H-purine-2,6(3H,7H)-dione (Compound 137).

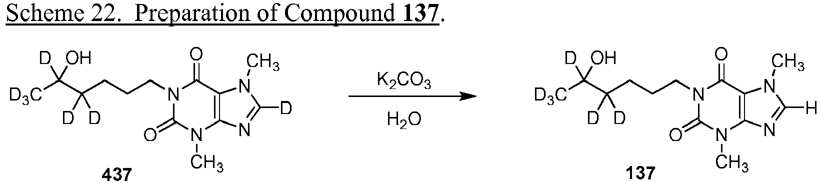

Synthesis of (±)-1-(4,4,5,6,6,6-d₆-5-Hydroxyhexyl)-3,7-dimethyl-1*H*-purine-2,6(3*H*,7*H*)-dione (Compound **137).** Compound **437** (560 mg, approximately 2 mmol, see Example 11) was stirred with K₂CO₃ (270 mg, 2 mmol) in water (10 mL). The mixture was heated at 120-130 °C to give a clear solution and was heated overnight. The solution was extracted with CH₂Cl₂ (1 x 50 mL, 2 x 20 mL) and the CH₂Cl₂ solution was dried (Na₂SO₄) and filtered. After removal of solvent, the solid was stirred with K₂CO₃ (140 mg, 1 mmol) in water (10 mL) and was heated overnight as above to ensure complete deuterium-to-hydrogen exchange. After extraction with CH₂Cl₂ (1 x 50 mL, 2 x 20 mL), the CH₂Cl₂ solution was dried (Na₂SO₄), filtered and concentrated. The crude product was purified by chromatography on silica gel eluting with 2-3% MeOH/CH₂Cl₂ to give 480 mg (86%) of 137.
HPLC (method: Zorbax 4.6x50 mm SB-Aq 3.5 µm column - gradient method 2-98% ACN + 0.1% formic acid in 6.0 min with MSD in ESI positive mode; 0.63 mL/min; Wavelength: 254 nm): retention time: 2.51 min; 98.7% purity. MS (M+H): 287.1; (M+Na): 309.0.
In general, any compound of the invention A having a group 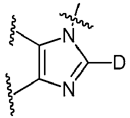 may be further converted to a compound of the invention having the same structure except for having a group 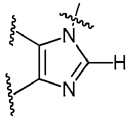 by treating with a suitable base and a proton source, such as water.

### Example 20 (reference). Synthesis of (R)-1-(4,4,5,6,6,6-d₆-5-Hydroxyhexyl)-3,7-dimethyl-1H-purine-2,6(3H,7H)-dione (Compound 137(R)).

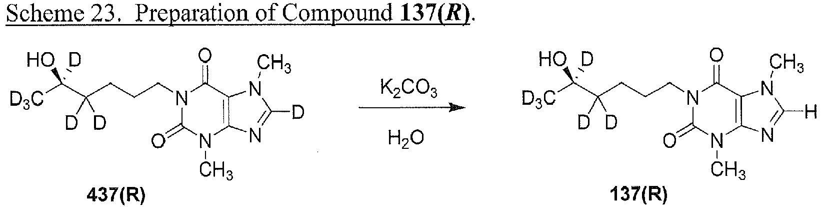

Synthesis of (*R*)-1-(4,4,5,6,6,6-d₆-5-Hydroxyhexyl)-3,7-dimethyl-1*H*-purine-2,6(3*H*,7*H*)-dione (Compound **137(*R*)).** A solution of **437(*R*)** (650 mg, 2.26 mmol, see Example 12) and K₂CO₃ (320 mg, 2.3 mmol) in water (40 mL) was heated at 110 °C (bath temperature) for 26 hours. The solution was concentrated to dryness, redissolved in water (30 mL) and heated to 100 °C for a further 6 hours. After cooling to ambient temperature the solution was extracted with CH₂Cl₂ (4 x 50 mL). The organic solution was dried (Na₂SO₄), filtered, concentrated, then dried under vacuum to afford 565 mg of **137(*R*)** as an off-white solid.
¹H-NMR (300 MHz, CDCl₃): δ 1.38-1.48 (m, 2H), 1.64-1.72 (m, 3H), 3.58 (s, 3H), 3.99 (d, *J*=0.5, 3H), 4.02 (t, *J*=7.4, 2H), 7.51 (d, *J*=0.6, 1H). ¹³C-NMR (75 MHz, CDCl₃): δ 22.65, 27.84, 29.71, 33.61, 41.13, 107.67, 141.43, 148.73, 151.50, 155.37. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 305 nm): retention time: 3.30 min; >99.9% purity. MS (M+H-H₂O): 269.4; (M+H): 287.1; (M+Na): 309.3. Elemental Analysis (C₁₃H₁₄D₆N₄O₃): Calculated: C=54.53, H=7.04, N=19.57. Found: C=54.43, H=6.93, N=19.44.

Alternative synthesis of (R)-1-(4,4,5,6,6,6-d₆-5-Hydrohexyl)-3,7-dimethyl-1H-purine-2,6(3H,7H)-dione (Compound **137(*R*))*.*** In a 3-L 3-necked RB flask, Compound 437(*R*) (100 g) was charged followed by water (1.0 L) and K₂CO₃ (0.25 equiv). The reaction mixture was heated to 80±5°C. and monitored by ¹H NMR. The reaction was complete after 24 hours and worked up after 65 hours. The resulting product was extracted with three times with EtOAc and the solid products from the three extractions combined and re-dissolved in 5 volumes of EtOAc at 60-65°C. n-heptane (5.5 vol.) was added at 60-65°C over 15 minutes and cooled to 20°C over night (16 hrs). The slurry was filtered and the wet cake was washed with n-heptane (2x1 vol. to afford product Compound 137(*R*) after drying at 40-50°C. A total of 92.4 g of Compound 137(*R*) was isolated. HPLC purity was 99.92% (AUC) and chiral selectivity was 100% to "S" enantiomer. The ¹H NMR analysis showed 99.2% of "H" at the 8-position in the 3,4,5,7-tetrahydro-1H-purine-2,6-dione ring and 99.4% of "D" at the methyl position.

### Example 21 (reference). Synthesis of (S)-1-(4,4,5,6,6,6-d₆-5-Hydroxyhexyl)-3,7-dimethyl-1H-purine-2,6(3H,7H)-dione (Compound 137(S)).

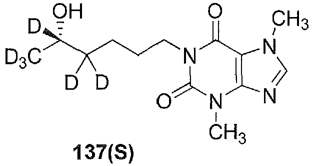

Following the same general method as for the synthesis of Compound **137(*R*)** in Example 20 above, a portion of Compound **437(*S*)** (see Example 12) was converted to 310 mg of Compound **137(*S*).**
¹H-NMR (300 MHz, CDCl₃): δ 1.36-1.45 (m, 2H), 1.62 (s, 1H), 1.64-1.74 (m, 2H), 3.58 (s, 3H), 3.99 (s, 3H), 4.02 (t, *J*=7.3, 2H), 7.50 (s, 1H). ¹³C-NMR (75 MHz, CDCl₃): δ 23.05, 28.24, 30.07, 33.95, 41.49, 107.92, 141.57, 148.93, 151.68, 155.53. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 305 nm): retention time: 3.34 min; 99.6% purity. MS (M+H-H₂O): 269.1; (M+H): 287.1; (M+Na): 309.3. Elemental Analysis (C₁₃H₁₄D₆N₄O₃): Calculated: C=54.53, H=7.04, N=19.57. Found: C=54.71, H=7.28, N=19.53.

Notable in the ¹H-NMR spectrum of **137*(S)*** was the absence of a peak at around 3.80 ppm, indicating an absence of hydrogen at the methinyl hydroxyl position.

### Example 22. Synthesis of (±)-1-(4,4,6,6,6-d₅-5-Hydroxyhexyl)-3,7-dimethyl-1H-purine-2,6(3H,7H)-dione (Compound 121).

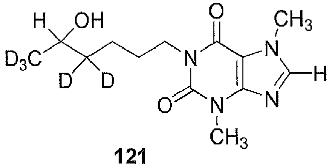

Following the same general method as for the synthesis of Compound **137** in Example 19 above, a portion of Compound **421** (see Example 17) was converted to 2.1 g of Compound **121.**
¹H-NMR (300 MHz, CDCl₃): δ 1.41-1.48 (m, 2H), 1.64-1.72 (m, 2H), 1.85 (bs, 1H), 3.58 (s, 3H), 3.79 (s, 1H), 3.99 (d, *J*=0.5*,* 3H), 4.02 (t, *J*=7.3, 2H), 7.52 (d, *J*=0.6, 1H). ¹³C-NMR (75 MHz, CDCl₃): δ 22.69, 27.82, 29.70, 33.61, 41.14, 67.55, 107.66, 141.44, 148.72, 151.49, 155.35. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 305 nm): retention time: 3.31 min; 99.3% purity. MS (M+H-H₂O): 268.2; (M+H): 286.2; (M+Na): 308.1. Elemental Analysis (C₁₃H₁₅D₅N₄O₃): Calculated: C=54.72, H=7.07, N=19.64. Found: C=54.75, H=6.85, N=19.54.

### Example 24. S-1-(4,4,6,6,6-d₅-5-Hydroxyhexyl)-3,7-dimethyl-1H-purine-2,6(3H,7H)-dione (Compound 121(S)).

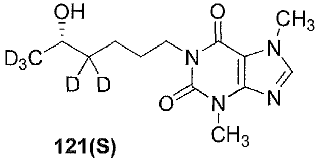

Following the same general method as for the synthesis of Compound **137(*R*)** in Example 20 above, a portion of Compound **421(*S*)** (see Example 18) was converted to 590 mg of Compound **121(*S*).**
¹H-NMR (300 MHz, CDCl₃): δ 1.37-1.48 (m, 2H), 1.64-1.73 (m, 2H), 1.86 (bs, 0.5H), 3.58 (s, 3H), 3.79 (s, 1H), 3.99 (d, *J*=0.6, 3H), 4.02 (t, *J*=7.4, 2H), 7.52 (d, *J*=0.7*,* 1H). ¹³C-NMR (75 MHz, CDCl₃): δ 22.70, 27.84, 29.71, 33.62, 41.14, 67.59, 107.67, 141.43, 148.73, 151.50, 155.37. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 305 nm): retention time: 3.37 min; 99.5% purity. Chiral HPLC (method: Chiralpak AD 25 cm column - isocratic method 78% hexane/ 22% isopropanol/0.1% diethylamine for 40 minutes at 1.00 mL/min; Wavelength: 254 nm): retention time: 25.20 min (expected for R enantiomer); 28.78 min (S enantiomer); >99% *ee* purity. MS (M+H-H₂O): 268.2; (M+H): 286.2; (M+Na): 308.1. Elemental Analysis (C₁₃H₁₅D₅N₄O₃): Calculated: C=54.72, H=7.07, N=19.64. Found: C=54.77, H=7.13, N=19.59.

Alternatively, Compound **121(*S*)** is synthesized from pentoxifylline **(58)** in a two step method according to Scheme 24: 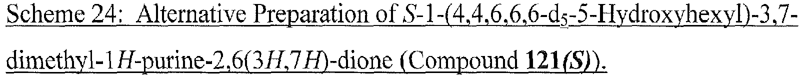 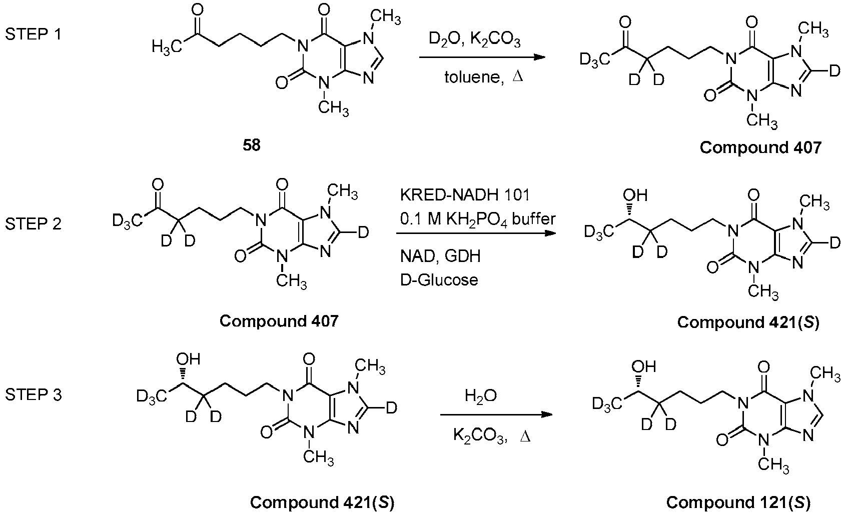
**Step 1. Compound 407.** Pentoxifylline **(58;** 1 mol equiv) was combined with toluene (20 volumes). To the mixture was added D₂O (1.5 volumes) and potassium carbonate (0.25 equiv) and the mixture was heated to reflux (ca. 87°C) for 3-4 hrs. The mixture was cooled to 40-50°C and the aqueous layer was removed. To the remaining toluene solution was added D₂O (1.5 volumes) and potassium carbonate (0.25 equiv) and the mixture was heated to reflux (ca. 87°C) for 3-4 hrs. The mixture was cooled to 40-50°C and the aqueous layer was removed. To the remaining toluene solution was added D₂O (1.5 volumes) and potassium carbonate (0.25 equiv) and the mixture was heated to reflux (ca. 87°C) for 3-4 hrs. The mixture was cooled to 40-50°C and the aqueous layer was removed. The organic layer was concentrated to ca. 5 volumes below 45°C, was cooled to 20-25°C and then heptane (1 volume) was added, followed by stirring at 20-25°C for 30 min. The slurry was filtered and washed with heptane, followed by drying in vacuo at 40-50°C to a constant weight. The yield of Compound **407** was approximately 90%.
**Step 2. Compound 421(*S*).** A 3-necked 12-L RB flask equipped with a heating mantle, a J-Kem thermocouple, a mechanical stirrer, and a pH probe was charged with glucose (547.5g, Aldrich lot # 088K0039) followed by 3.47 L of 0.1M KH₂PO₄, pH = 7.0 ("Buffer"; 9.5 vol). The reaction mixture was stirred to dissolve all solids. A mixture of Compound **407** (365 g) in Buffer (2.92 L) was added and the container was rinsed with Buffer (1.28 L). The rinse was added to the reactor. Initially, the reaction mixture was a very thin milky suspension. A solution of KRED-NADH-101 (3.65 g, CODEXIS lot # 1021908WW), NAD (2.19 g, SPECTRUM lot # YA0655), GDH (365 mg, CODEXIS lot # 22016700017) in Buffer (1.46 L) was charged to the reactor. The container was rinsed with Buffer (2 x 0.91 L) and the rinses were added to the reactor. The reaction mixture was warmed to 20-30°C and monitored by a pH meter. The reaction mixture turned clear after 30 minutes. The pH of the reaction mixture was maintained between 6.50 and 6.90 by adding 4M KOH solution drop-wise as needed. The reaction was monitored by HPLC and was complete after 5 hours with 99.97% conversion by HPLC. The reaction mixture was stirred at 20-25°C overnight and warmed to 30°C for the work-up.
   Sodium chloride (1.825 kg) was added to the reaction mixture and dissolved completely after stirring for 15 minutes. The batch was extracted with EtOAc (10 vol). The organic phase contained a thin solid gel, which collapsed into a slimy separate phase between the aqueous and organic layers immediately when agitated slightly. The slime could be retained on a paper filter but formed a thin impermeable layer that prevented flow through the filter. It was observed on a sample that a small amount of filter aid (celite) easily adsorbed the slime. The aqueous layer was charged back to the reactor and extracted with EtOAc (10 vol). Filter aid (100 g) was charged to the reactor to absorb the slime. The batch was filtered (less than one hour) and the organic layer was collected. The aqueous layer was then extracted with EtOAc (2 x 5 vol) without any problems (no further slime or emulsion was observed). The combined organic extracts were concentrated to ca. 10 volume and polish filtered to remove a small amount of the inorganic solids. The filtrate was concentrated further to ca. 5 volumes and product solids were precipitated. n-heptane (8 vol) was added to the slurry (at 40-60°C) over 30 minutes. The slurry was stirred overnight at 20-25°C and filtered. The filter cake was washed with n-heptane (2 x 1 vol). The wet cake (370 g) was dried at 40-50°C over the weekend to afford Compound 421(*S*) as a white solid (332.0 g, 90.0% yield). The filtrate was concentrated followed by precipitation with heptane to afford a second crop of Compound **421(*S*)** (7.1 g, 1.9% yield). In order to check the mass balance of the product, the aqueous layer was extracted again with EtOAc (10 vol) and afforded only 4.8 g of Compound **421(*S*)** (1.3% yield) of product as a white solid. The combined mother liquor was concentrated to afford 2.0 g of Compound **421(*S*)** as a yellow solid (0.5% yield). The isolated product was a very high quality (100% purity by HPLC) and a single enantiomer (100/0 S/R% by chiral HPLC) from the main lot with 99.5% "D" incorporation at the methyl position by ¹H NMR.

### Step 2 (alternative procedure):

**[1]** A 12-L 3-necked RB flask equipped with a heating mantle, a J-Kem thermocouple, a mechanical stirrer, a reflux condenser, and a pH probe was charged with CRED A131 (9.5 g, ALMAC lot # IM-1311-061-1) and 2 L of buffer solution (0.1M KH2PO4, pH = 7.0, same as below). The reaction mixture was stirred to dissolve all solids. A solution of glucose (558 g, Aldrich lot # 088K0039) in buffer (2 L) was added in one portion followed by a solution of NAD (19.25 g, Spectrum lot # YA0655) in buffer (500 mL), and a solution of GDH (1.5 g, ALMAC lot # IM-1311-131-1) in buffer (500 mL). The initial reaction mixture was pH 6.98. A mixture of Compound **407** in buffer (3 L) at 30 °C was added to the reaction mixture and the container was rinsed with buffer (1.6 L). The rinse was charged to the reactor. The pH of the reaction mixture was 6.99. The reaction mixture was warmed to 30°C and monitored by pH meter. The reaction temperature was kept at 29.0 to 31.5°C and the pH of the reaction mixture was kept between pH 6.93 and pH 7.02 by adding 4M KOH solution drop-wise as needed. The reaction was complete after 22 hours with 99.96% conversion as determined by HPLC. The chiral HPLC analysis of the resulting product showed the chiral selectivity was 99.85% to the desired S-alcohol.
[2] The reaction mixture was mixed with NaCl (2 kg) and extracted with EtOAc (1 x 4 L and 3 x 2 L). During the first extraction, a rag layer was formed and the reaction mixture was filtered through a celite pad. No further issues with phase separation were encountered after the filtration. The combined organic extracts were concentrated to about 1.5 L at 50-60°C and n-heptane (2 L) was added to precipitate the solids. The slurry was cooled to 20° C and filtered. The flask was rinsed with filtrate to complete the transfer. The filter cake was washed with n-heptane (2 x 500 mL) and dried over the weekend at 40-50° C to afford Compound **421(*S*)** (366 g, 94% yield). The product was analyzed by HPLC (99.95% purity), chiral HPLC (99.88/0.12 S/R), and ¹H NMR (99.5% "D" incorporation at the methyl position). **Step 3. Compound 121(*S*).** In a 3-L 3-necked RB flask, Compound **421(*S*)** (100 g) was charged followed by water (1.0 L) and K₂CO₃ (0.25 equiv). The reaction mixture was heated to 80±5°C. and monitored by ¹H NMR. The reaction was complete after 24 hours and worked up after 65 hours. The resulting product was extracted with three times with EtOAc and the solid products from the three extractions combined and re-dissolved in 5 volumes of EtOAc at 60-65°C. n-heptane (5.5 vol.) was added at 60-65°C over 15 minutes and cooled to 20°C over night (16 hrs). The slurry was filtered and the wet cake was washed with n-heptane (2x1 vol. to afford product Compound **121(*S*)** after drying at 40-50°C. A total of 92.4 g of Compound **121(*S*)** was isolated. HPLC purity was 99.92% (AUC) and chiral selectivity was 100% to "S" enantiomer. The ¹H NMR analysis showed 99.2% of "H" at the 8-position in the 3,4,5,7-tetrahydro-1H-purine-2,6-dione ring and 99.4% of "D" at the methyl position.

### Example 25 (reference). Synthesis of 3,7-Dimethyl-1-(4,4,6,6,6-d₅-5-oxohexyl)-1H-purine-2,6(3H,7H)-dione Compound 107).

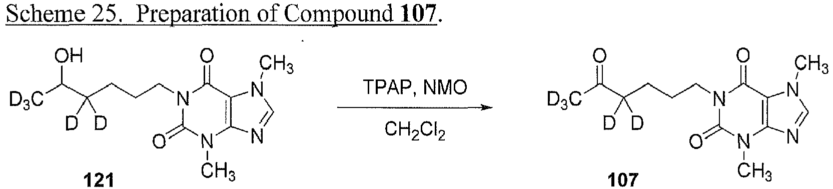

Synthesis of 3,7-Dimethyl-1-(4,4,6,6,6-d₅-5-oxohexyl)-1*H*-purine-2,6(3*H*,7*H*)-dione (Compound **107).** Compound **121** (0.49 g, 1.72 mmol, see Example 22) and N-methylmorpholine N-oxide "NMO" (301 mg, 2.58 mmol) were dissolved in CH₂Cl₂ (20 mL). Tetrapropylammonium perruthenate "TPAP" (27 mg, 0.086 mmol) was added and the solution was stirred for 2.5 hours at ambient temperature. TLC (EtOAc) showed the reaction was complete. The reaction was concentrated and purified by silica gel chromatography eluting with EtOAc. The material was dried in a vacuum oven (50 °C) for 4 hours to afford 400 mg (82%) of Compound **107.** The material was further purified by crystallization (EtOAc/heptane) to give 320 mg of **107.** NMR and LCMS analysis indicated no loss of deuterium.
¹H-NMR (300 MHz, CDCl₃): δ 1.64-1.70 (m, 4H), 3.57 (s, 3H), 3.99 (d, *J*=0.6, 3H), 4.01-4.04 (m, 2H), 7.51 (d, *J*=0.6, 1H). ¹³C-NMR (75 MHz, CDCl₃): δ 20.82, 27.38, 29.69, 33.61, 40.80, 107.75, 141.42, 148.76, 151.46, 155.26. HPLC (method: Waters Atlantis T3 2.1 x 50 mm 3 µm C18-RP column - gradient method 5-95% ACN + 0.1% formic acid in 14 minutes (1.0 mL/min) with 4 minute hold at 95% ACN; Wavelength: 305 nm): retention time: 3.28 min; >99.9% purity. MS (M+H): 284.1; (M+Na): 306.0.

### BIOLOGICAL EVALUATION

### Example 40. Pharmacokinetic Study of 435(S) and other representative compounds in Chimps After Oral Dosing.

**435*(S)**** and representative compounds **121*(S),* 137*(S)**, 421*(S)**** and **437*(S)**** were separately dissolved in warm (65 °C) water at a concentration [[of 10 mg/mL]]. The same protocol described for Example 39 was then followed for each representative compound.
*not part of the present invention.

**Table 17 i) - iv). Pharmacokinetic Results Following Oral Administration in Chimps.**

| i) | | |
|---|---|---|
| | AUC₀₋₁₂ (hr*ng/mL) | |
| Compound(s) Measured^{a} | Male | Female |
| **435(*S*)** | 354 | 133 |
| **121*(S)*** | 304 | 105 |
| | | |
| **435(*S*)** + **409** | 715 | 282 |
| **121*(S)*+107** | 553 | 224 |
| | | |
| **Deuterated M5 (M5a)** | 585 | 670 |
| **M5** | 630 | 653 |

| ii) | | |
|---|---|---|
| | AUC₀₋₁₂ (hr*ng/mL) | |
| Compound(s) Measured^{a} | Male | Female |
| **435(*S*)** | 100 | 243 |
| **137*(S)*** | 63 | 163 |
| | | |
| **435(*S*)** + **409** | 195 | 435 |
| **137*(S)*+107** | 127 | 381 |
| | | |
| **Deuterated M5 (M5a)** | 719 | 754 |
| **M5** | 539 | 635 |

| iii) | | |
|---|---|---|
| | AUC₀₋₁₂ (hr*ng/mL) | |
| Compound(s) Measured^{a} | Male | Female |
| **435(*S*)** | 881 | 947 |
| **421(*S*)** | 743 | 634 |
| | | |
| **435(*S*)** + **409** | 1130 | 1000 |
| **421(*S*)+407** | 979 | 834 |
| | | |
| **Deuterated M5 (M5a)** | 500 | 376 |
| **Deuterated M5 (M5b)** | 447 | 350 |

| iv) | | |
|---|---|---|
| | AUC₀₋₁₂ (hr*ng/mL) | |
| Compound(s) Measured^{a} | Male | Female |
| **435(*S*)** | 686 | 1140 |
| **437(*S*)** | 757 | 1178 |
| | | |
| **435(*S*)** + **409** | 876 | 1654 |
| **437*(S)*+407** | 947 | 1662 |
| | | |
| **Deuterated M5 (M5a)** | 562 | 306 |
| **Deuterated M5 (M5b)** | 627 | 416 |

| | | |
|---|---|---|
| a) Mass observed via LC-MS/MS. | | |

Table 17 i)-iv) shows the results of oral administration of **435(*S*)** and of compounds **121*(S),* 137*(S)*, 421*(S)*** and **437*(S)*,** respectively, in chimps. For each chimp, the values of AUC₀₋₁₂ for each of **121*(S),* 137*(S)*, 421*(S)*** and **437(*S*)** are comparable to the values of AUC₀₋₁₂ for of **435*(S)*.** Similarly, the sum of AUC₀₋₁₂ values for **435*(S)*** and **409** is comparable to the sums of AUC₀₋₁₂ values for each of **121*(S),* 137*(S)*, 421*(S)*** and **437*(S)*** and their respective ketone metabolites **107, 107, 407** and **407.**

Finally, comparable values were also found for **435*(S)*** metabolite **M5a** and the corresponding metabolites of **121*(S),* 137*(S)*, 421*(S)*** and **437*(S)*,** i.e., **M5, M5, M5b** and **M5b** (shown below).

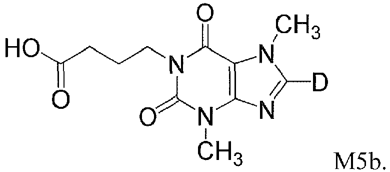

Without further description, it is believed that one of ordinary skill in the art can, using the preceding description and the illustrative examples, make and utilize the compounds of the present invention and practice the claimed medical uses. It should be understood that the foregoing discussion and examples merely present a detailed description of certain preferred embodiments. It will be apparent to those of ordinary skill in the art that various modifications can be made without departing from the scope of the invention.

## Claims

1. A compound of the following formula: 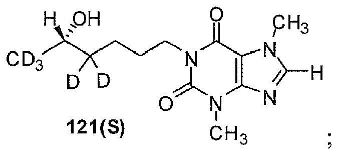 or a pharmaceutically acceptable salt thereof, wherein the isotopic enrichment factor for each designated deuterium atom is at least 5000.

2. The compound of claim 1, wherein any atom not designated as deuterium is present at its natural isotopic abundance.

3. The compound of Claim 2, wherein the isotopic enrichment factor for each designated deuterium atom is at least 6000.

4. The compound of Claim 2, wherein the isotopic enrichment factor for each designated deuterium atom is at least 6600.

5. The compound of any of claims 1 to 4, wherein the compound comprises less than 10% of the other stereoisomer.

6. The compound of Claim 5, wherein the compound comprises less than 5% of the other stereoisomer.

7. A pharmaceutical composition comprising a compound of any of claims 1 to 6 and a pharmaceutically acceptable carrier.

8. A compound of any one of claims 1 to 6 for use in treating a disease or condition selected from diabetic nephropathy, hypertensive nephropathy or intermittent claudication on the basis of chronic occlusive arterial disease of the limbs.

9. A compound of any one of claims 1 to 6 for use in treating chronic kidney disease.

10. The compound for use of claim 9 wherein the chronic kidney disease is glomerulonephritis, focal segmental glomerulosclerosis, nephrotic syndrome, reflux uropathy, or polycystic kidney disease.

## Patentansprüche

1. Verbindung der folgenden Formel: 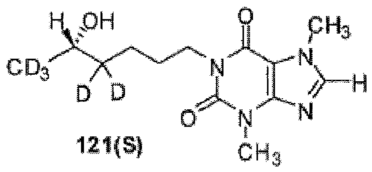 oder ein pharmazeutisch akzeptables Salz davon, wobei der Isotopenanreicherungsfaktor für jedes bezeichnete Deuteriumatom mindestens 5000 beträgt.

2. Verbindung nach Anspruch 1, wobei jedes Atom, das nicht als Deuterium bezeichnet ist, in seiner natürlichen Isotopenhäufigkeit vorliegt.

3. Verbindung nach Anspruch 2, wobei der Isotopenanreicherungsfaktor für jedes bezeichnete Deuteriumatom mindestens 6000 beträgt.

4. Verbindung nach Anspruch 2, wobei der Isotopenanreicherungsfaktor für jedes bezeichnete Deuteriumatom mindestens 6600 beträgt.

5. Verbindung nach einem der Ansprüche 1 bis 4, wobei die Verbindung weniger als 10% des anderen Stereoisomers umfasst.

6. Verbindung nach Anspruch 5, wobei die Verbindung weniger als 5% des anderen Stereoisomers umfasst.

7. Pharmazeutische Zusammensetzung, umfassend eine Verbindung nach einem der Ansprüche 1 bis 6 und einen pharmazeutisch akzeptablen Träger.

8. Verbindung nach einem der Ansprüche 1 bis 6 zur Verwendung bei der Behandlung einer Krankheit oder eines Leidens ausgewählt aus diabetischer Nephropathie, hypertonischer Nephropathie oder Claudicatio intermittens auf der Basis einer chronischen arteriellen Verschlusskrankheit der Gliedmaßen.

9. Verbindung nach einem der Ansprüche 1 bis 6 zur Verwendung bei der Behandlung von chronischer Nierenkrankheit.

10. Verbindung zur Verwendung nach Anspruch 9, wobei es sich bei der chronischen Nierenkrankheit um Glomerulonephritis, fokalsegmentale Glomerulosklerose, nephrotisches Syndrom, Refluxuropathie oder polyzystische Nierenkrankheit handelt.

## Revendications

1. Composé de formule suivants : 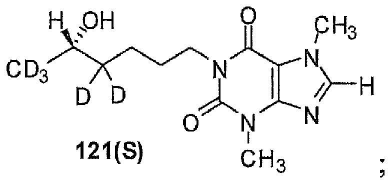 ou un sel pharmaceutiquement acceptable de celui-ci, dans lequel le facteur d'enrichissement isotopique pour chaque atome de deutérium désigné est d'au moins 5000.

2. Composé selon la revendication 1, dans lequel un atome quelconque non désigné comme deutérium est présent selon son abondance isotopique naturelle.

3. Composé selon la revendication 2, dans lequel le facteur d'enrichissement isotopique pour chaque atome de deutérium désigné est d'au moins 6000.

4. Composé selon la revendication 2, dans lequel le facteur d'enrichissement isotopique pour chaque atome de deutérium désigné est d'au moins 6600.

5. Composé selon l'une quelconque des revendications 1 à 4, dans lequel le composé comprend moins de 10% de l'autre stéréoisomère.

6. Composé selon la revendication 5, dans lequel le composé comprend moins de 5% de l'autre stéréoisomère.

7. Composition pharmaceutique comprenant un composé selon l'une quelconque des revendications 1 à 6 et un véhicule pharmaceutiquement acceptable.

8. Composé selon l'une quelconque des revendications 1 à 6, pour une utilisation dans le traitement d'une maladie ou condition choisie parmi la néphropathie diabétique, la néphropathie hypertensive ou la boiterie intermittente sur la base d'une artériopathie occlusive chronique des membres.

9. Composé selon l'une quelconque des revendications 1 à 6, pour une utilisation dans le traitement d'une maladie rénale chronique.

10. Composé pour une utilisation selon la revendication 9, dans lequel la maladie rénale chronique est la glomérulonéphrite, la glomérulosclérose segmentaire focale, le syndrome néphrotique, l'uropathie par reflux, ou la maladie polykystique des reins.
